# EUROPEAN PATENT SPECIFICATION

(11) **EP 3 758 851 B1**
(45) Date of publication and mention of the grant of the patent: **04.09.2024**
(21) Application number: 19758305.7
(22) Date of filing: 26.02.2019
(51) Int. Cl.: B04B 5/04, B04B 13/00, B04B 7/08, G01N 21/07, G01N 15/04, G01N 35/00, C12Q 1/68, G01N 21/45, G01N 21/64, G01N 21/03

(54) **INTEGRATED ROTOR DEVICES FOR AUTONOMOUS ANALYTICAL CENTRIFUGATION, INTEGRATED CELL DEVICES FOR AUTONOMOUS ANALYTICAL CENTRIFUGATION, AND METHODS OF ASSEMBLY AND OPERATION OF SAME**
INTEGRIERTE ROTORVORRICHTUNGEN FÜR AUTONOME ANALYTISCHE ZENTRIFUGATION, INTEGRIERTE ZELLVORRICHTUNGEN FÜR AUTONOME ANALYTISCHE ZENTRIFUGATION UND VERFAHREN ZUR MONTAGE UND ZUM BETRIEB DAVON
DISPOSITIFS DE ROTOR INTÉGRÉS POUR CENTRIFUGATION ANALYTIQUE AUTONOME, DISPOSITIFS DE CELLULE INTÉGRÉS POUR CENTRIFUGATION ANALYTIQUE AUTONOME, ET LEURS PROCÉDÉS D'ASSEMBLAGE ET DE FONCTIONNEMENT

(30) Priority: 26.02.2018 US 201862635514 P
(43) Date of publication of application: 06.01.2021
(73) Proprietor: Higher Order Technologies, LLC., Scituate MA 02066 (US)
(72) Inventor: SUCATO, Christopher A., Pleasant Valley, New York 12569 (US); DIPAOLA, Mario, Scituate, Massachusetts 02066 (US)
(74) Representative: Hannke Bittner & Partner mbB Regensburg
(86) International application number: PCT/US2019/019691
(87) International publication number: WO 2019/165478

(56) References cited:
- EP-A1- 2 484 391
- WO-A1-2016/160962
- US-A- 3 487 994
- US-A1- 2006 205 581
- US-A1- 2008 058 991
- US-A1- 2012 308 435
- US-A1- 2014 371 047
- US-A1- 2018 195 938

## Description

### BACKGROUND

Analytical Ultracentrifuge (AUC) instruments are centrifuges known for generating sedimentation data that can be deconvoluted for the determination of size distribution profiles in protein and other biological samples, for the purpose of evaluating the extent of aggregates or other impurities in fluid samples. An early example is disclosed in US Patent No. 1648369 wherein the fundamental components of an AUC instrument are described in detail. Later examples include US Patent Nos. 2340825, 3391597, 3487994, and 4226537, which present improvements to AUC instrument design and the introduction of improved supporting devices, including the AUC cell, which is assembled and inserted into specialized rotors that allow data collection via modules within the centrifuge.

A standard procedure for the operation of a conventional AUC instrument and its accessory hardware components is to first assemble the AUC cell, which is a layered collection of windows, sector-shaped sample chamber, and outer housing and screw rings. Specifically, a sector-shaped geometry is defined as the volume element of a cylindrical segment having the cross-sectional area of a circular sector (an area enclosed by two radii of a large circle, the bounding arc of the larger circle between the radii, and an arc of a smaller concentric circle of lesser radius). Once torqued so as to render the sample chamber fluid-tight, the fluid sample may be loaded into the chamber through small ports, which are also subsequently sealed. The AUC cell is placed into a rotor which is typically solid titanium with cylindrical cavities parallel to the axis of rotation and arranged symmetrically about said axis of rotation. The rotor is placed inside the AUC instrument and allowed to equilibrate to the desired temperature via thermostatting of the centrifuge chamber, controlled by refrigeration or heating elements which are located inside the centrifuge. The centrifuge is also programmed to accelerate the rotor to the desired centrifugal velocity and maintain the velocity for a fixed period.
WO 2016/160962 A1 shows A centrifuge, which includes: a motor having a rotor; an imaging system torsionally connected to the rotor; a sample holder torsionally connected to the imaging system; and a light source for illuminating the sample holder. The imaging system includes: a image sensor in optical communication with the sample holder; and a data link for transmitting image data. A centrifuge test includes: spinning a reservoir core sample in a sample holder of a rotor, wherein an imaging system is torsionally connected to the rotor; and collecting image data of the sample holder with the imaging system while spinning.
US 2014/371047 A1 shows a centrifuge rotor for measuring a characteristic of a sample subjected to centrifugation, the centrifuge rotor including: a rotor body; at least one sample holder for holding a sample; at least one on-board measurement device for measuring a characteristic of the sample held in the at least one sample holder during rotation of the rotor.
US 2006/205581 A1 shows a blood processing centrifuge comprising: a rotor having an axis of rotation and being controllably spun around the axis, a mechanism for processing whole blood within the rotor while spinning, a computer controlling blood processing operations, the computer being mounted to the rotor and spinning therewith.
EP 2 484 391 A1 discloses a blood separation systems and methods are provided for controlling the interface between separated blood components. The system includes a blood separation chamber configured to separate blood into first and second blood components and an outlet line for removing at least a portion of the first blood component from the blood separation chamber. A primary optical sensor assembly is associated with the blood separation chamber to directly monitor the interior of the blood separation chamber. A secondary optical sensor assembly is associated with the outlet line to monitor the first blood component in the outlet line. The system also includes a controller programmed to select between the primary optical sensor assembly and the secondary optical sensor assembly for monitoring contamination of the first blood component. The system is particularly advantageous for preventing contamination of separated plasma which is lipemic or hemolytic.

### SUMMARY

Embodiments relate to centrifugation, and, more specifically, to analytical centrifugation or ultracentrifugation systems and methods for detecting in fluid samples the degree of sedimentation of soluble molecules, aggregates, or particles. In some embodiments, detection can be performed in real time and continuously. In some embodiments, detection can be performed during the course of the sedimentation process. Some embodiments provide for an assembly of modules or sub-units for sample containment, illumination and detection. In some embodiments, supporting functions are integrated and confined within the geometry of a rotor. In some embodiments, supporting functions of the sample interrogation, data generation and data collection, as well as modulation and control of sample condition, are integrated into units, and, in some embodiments, assembled into structures, or cells that are affixed within the rotor. The resulting configuration can be rotated by an externally applied centrifugal force, such as that supplied by a standard floor-model centrifuge, ultracentrifuge, or table-top centrifuge, which are commonly available. In some embodiments, the resulting self-contained, integrated device can generate, store, and transmit data for completing an analytical centrifugation or ultracentrifugation experiment.

In an aspect an interrogation cell, comprising: a source of electromagnetic radiation at a first position, the source of electromagnetic radiation configured to emit electromagnetic radiation at one or more wavelengths; a sample region; and a detector at a second position, the detector configured to receive electromagnetic radiation that traverses at least a portion of the sample region, characterized in that the interrogation cell is dimensioned for positioning in a rotor cavity of a centrifuge rotor.

In an embodiment, the rotor comprises a central opening.

In an embodiment, the central opening is aligned with the axis of rotation.

In an embodiment, the rotor is constructed and arranged to mate with a spindle.

In an embodiment, the spindle is aligned with the axis of rotation.

In an embodiment, the spindle comprises a spindle of a centrifuge.

In an embodiment, the rotor is oriented symmetrically about the axis of rotation.

In an embodiment, the rotor comprises one or more sub-units configured to be stacked in a vertical direction of extension along the axis of rotation.

In an embodiment, neighboring ones of the one or more stacked sub-units are coupled to each other at a threaded interface.

In an embodiment, one or more of the one or more stacked sub-units are coupled with a bolt-through configuration.

In an embodiment, a gasket is positioned between one or more of the one or more stacked sub-units.

In an embodiment, the gasket is constructed and arranged to form a sealed region.

In an embodiment, the sealed region is sealed from an ambient region external to the sealed region.

In an embodiment, one or more of the one or more sub-units are coupled to each other with a tongue-in-groove structure.

In an embodiment, one or more of the one or more stacked sub-units comprise high-strength materials.

In an embodiment, the high-strength material comprises titanium.

In an embodiment, the high-strength material comprises an alloy material.

In an embodiment, the high-strength material comprises a composite material.

In an embodiment, the high-strength material comprises a material including a carbon fiber material.

In an embodiment, the sample region comprises a sector shape.

In an embodiment, the sample region comprises a sample chamber.

In an embodiment, the sample chamber comprises a removable liner.

In an embodiment, the first position and the second position are at the sample chamber and wherein the sample chamber comprises the source of electromagnetic radiation and the detector.

In an embodiment, the sample region comprises an open top and an open bottom.

In an embodiment, the sample region is coupled to one or more neighboring sub-units.

In an embodiment, a portion of the electromagnetic radiation emitted from the source of electromagnetic radiation is incident on the detector.

In an embodiment, the electromagnetic radiation is directed toward the sample region.

In an embodiment, the detector is arranged to detect electromagnetic radiation that traverses a portion of the sample region.

In an embodiment, the sample region, the source of electromagnetic radiation, and the detector are arranged along an axis that is parallel to the axis of rotation.

In an embodiment, the source of electromagnetic radiation is positioned at a first of the one or more stacked sub-units.

In an embodiment, the sample region is positioned at a second of the one or more stacked sub-units.

In an embodiment, the detector is positioned at a third of the one or more stacked sub-units.

In an embodiment, the system further comprises an alignment mechanism that aligns the first, second and third of the one or more stacked sub-units so that the source of electromagnetic radiation, the sample region and the detector being aligned in a vertical direction along an axis of interrogation that is parallel to the axis of rotation.

In an embodiment, a portion of the electromagnetic radiation emitted from the source of electromagnetic radiation traverses a portion of the sample chamber.

In an embodiment, one or more wavelengths of the electromagnetic radiation is incident at the sample region.

In an embodiment, electromagnetic radiation propagates through the sample region to the detector.

In an embodiment, electromagnetic radiation triggers a reaction of material at the sample region.

In an embodiment, the detector collects electromagnetic radiation emitted from the sample region and the electromagnetic radiation emitted from the sample region comprises one or more wavelengths that are different than the one or more wavelengths emitted from the source of electromagnetic radiation.

In an embodiment, the wavelengths of the electromagnetic radiation emitted from the sample region are greater than the one or more wavelengths emitted from the source of electromagnetic radiation.

In an embodiment, the wavelengths of the electromagnetic radiation emitted from the sample region are less than the one or more wavelengths emitted from the source of electromagnetic radiation.

In an embodiment, the electromagnetic radiation triggers a photophysical interaction.

In an embodiment, the photophysical interaction comprises excitation of molecules present in the sample region to energy levels above a ground state of the sample molecules.

In an embodiment, the excitation of the energy levels consists of at least one of excitation of electronic energy levels, excitation of vibrational energy levels or excitation of rotational energy levels.

In an embodiment, the source of electromagnetic radiation is one of a plurality of sources of electromagnetic radiation that is optically coupled with the detector.

In an embodiment, the source of electromagnetic radiation is coupled to a circuit board.

In an embodiment, the rotor comprises one or more sub-units configured to be stacked in a vertical direction of extension along the axis of rotation and wherein a topmost sub-unit of the one or more sub-units comprises a source of electromagnetic radiation coupled to a circuit board.

In an embodiment, the rotor comprises one or more sub-units configured to be stacked in a vertical direction of extension along the axis of rotation and wherein a topmost sub-unit of the one or more sub-units comprises a plurality of sources of electromagnetic radiation, wherein the sample region comprises a plurality of sample regions and wherein the detector comprises a plurality of detectors, each source of electromagnetic radiation being optically coupled to a corresponding detector through an optical path that includes a corresponding sample region.

In an embodiment, the source of electromagnetic radiation is a single light emitting diode (LED).

In an embodiment, the source of electromagnetic radiation comprises an array of light emitting diodes (LEDs).

In an embodiment, the source of electromagnetic radiation comprises a laser diode.

In an embodiment, the source of electromagnetic radiation comprises and array of laser diodes.

In an embodiment, the detector comprises an array detector.

In an embodiment, the detector comprises a point detector.

In an embodiment, the detector comprises a CCD array.

In an embodiment, the detector comprises a photodiode.

In an embodiment, the detector periodically samples incident electromagnetic radiation.

In an embodiment, the system further comprises a storage that stores information collected by the detector.

In an embodiment, the system further comprises a transmitter that transmits the stored information stored by the storage to a receiver.

In an embodiment, the transmitter transmits the stored information as the rotor is rotating about the axis of rotation.

In an embodiment, the system transmits the stored information when the rotor is stationary.

In an embodiment, the system is configured to measure light absorbance of the sample region based on the stored information collected by the detector.

In an embodiment, the system is configured to provide spectroscopic information of the sample region based on the stored information collected by the detector.

In an embodiment, the system is configured to provide hyperspectral image data of the sample region based on the stored information collected by the detector.

In an embodiment, the system is configured to provide Schlieren images of the sample region based on the stored information collected by the detector.

In an embodiment, the system is configured to provide fluorescence images of the sample region based on the stored information collected by the detector.

In an embodiment, the system is configured to provide quantitative fluorescence emission data of the sample region based on the stored information collected by the detector.

In an embodiment, the quantitative fluorescence emission data comprises a spatial arrangement of the sample region.

In an embodiment, the system further comprises a Fabry-Perot interferometer optically coupled with the source of electromagnetic radiation and the detector.

In an embodiment, the system further comprises one or more optical filters positioned between the source of electromagnetic radiation and the detector.

In an embodiment, the system further comprises one or more optical lenses positioned between the source of electromagnetic radiation and the detector.

In an embodiment, the system further comprises one or more mirrors positioned between the source of electromagnetic radiation and the detector.

In an embodiment, the system further comprises one or more optical diffusers positioned between the source of electromagnetic radiation and the detector.

In an embodiment, the system further comprises one or more optical collimators positioned between the source of electromagnetic radiation and the detector.

In an embodiment, one or more of the one or more optical collimators comprises at least one self-collimating photonic crystal.

In an embodiment, one or more of the one or more optical collimators comprises at least one micro-Fresnel lens.

In an embodiment, the system further comprises one or more optical lenses positioned between the source of electromagnetic radiation and the detector wherein the one or more optical lenses are positioned relative to the sample region and the detector such that the detector detects an image of a plane at the sample region.

In an embodiment, the one or more lenses are positioned below the sample region.

In an embodiment, the system further comprises an elongated edge constructed and arranged to block a portion of the light incident on the detector.

In an embodiment, the system further comprises an iris constructed and arranged to block a portion of the light incident on the detector.

In an embodiment, the system further comprises a beamsplitter system constructed and arranged to direct electromagnetic radiation from the source of electromagnetic radiation to a plurality of locations.

In an embodiment, the beamsplitter system comprises at least one mirror.

In an embodiment, the beamsplitter system comprises at least one filter.

In an embodiment, the beamsplitter system comprises at least one lens.

In an embodiment, the beamsplitter system is constructed and arranged such that electromagnetic radiation emitted from a first sub-unit of the rotor is optically coupled to a detector on a second sub-unit of the rotor different than the first sub-unit.

In an embodiment, the rotor comprises one or more sub-units configured to be stacked in a vertical direction of extension along the axis of rotation and wherein one or more of the one or more stacked sub-units are electrically connected with one or more connectors.

In an embodiment, the one or more connectors are configured to transfer information.

In an embodiment, the one or more connectors are configured to transfer power.

In an embodiment, one or more of the one or more stacked sub-units are electrically connected with USB ports.

In an embodiment, one or more of the one or more stacked sub-units are electrically connected with micro-USB ports.

In an embodiment, one or more of the one or more stacked sub-units are electrically connected with VGA ports.

In an embodiment, one or more of the one or more stacked sub-units are electrically connected with D-type connectors.

In an embodiment, the system further comprises a transmitter configured to transmit information from the detector.

In an embodiment, the transmitter is configured to transmit the information in an encrypted format.

In an embodiment, the transmitter is configured to transmit the information to a cloud-based storage system.

In an embodiment, the transmitter is configured to transmit information wirelessly.

In an embodiment, the transmitter comprises an antenna.

In an embodiment, the transmitter is configured to transmit the information optically.

In an embodiment, the transmitter comprises a cable connection.

In an embodiment, the cable connection comprises an electrical connection.

In an embodiment, the cable connection comprises fiber optics.

In an embodiment, the cable connection comprises connection to a circuit board.

In an embodiment, the cable connection comprises connection to an on-board memory.

In an embodiment, the on-board memory comprises a device selected from the group consisting of: a random access memory (RAM) device, a read-only memory (ROM) device, a solid-state memory (SSD) device, an SD memory card, or a micro-SD memory card.

In an embodiment, the transmitter is positioned at a bottom portion of the rotor.

In an embodiment, the rotor comprises a central opening aligned with the axis of rotation.

In an embodiment, the transmitter is constructed and arranged to extend through the central opening.

In an embodiment, the transmitter extends from the bottom of the rotor to the top of the rotor.

In an embodiment, the transmitter comprises one of a plurality of transmitters.

In an embodiment, the transmitter comprises a battery.

In an embodiment, the system further comprises a recharging mechanism that recharges the battery, the recharging mechanism converting rotational energy from the rotation of the rotor into electrical current.

In an embodiment, the recharging mechanism comprises an electric generator comprising a planetary gear configuration constructed and arranged to facilitate relative internal rotation in a rotor-stator pairing.

In an embodiment, the system further comprises an on-board memory at the rotor.

In an embodiment, the on-board memory comprises a device selected from the group consisting of: a random access memory (RAM) device, a read-only memory (ROM) device, a solid-state memory (SSD) device, an SD memory card, or a micro-SD memory card.

In an embodiment, the system further comprises a temperature control system configured to modify a temperature of the sample region.

In an embodiment, the temperature control system is configured to maintain the temperature of the sample region.

In an embodiment, maintaining the temperature comprises heating the sample region.

In an embodiment, maintaining the temperature comprises cooling the sample region.

In an embodiment, the temperature control system circulates thermally conductive material at the sample region.

In an embodiment, the temperature control system comprises a pumping system to circulate the thermally conductive material.

In an embodiment, the temperature control system heats the sample region.

In an embodiment, the temperature control system cools the sample region.

In an embodiment, the temperature control system circulates thermally conductive material through channels at one or more sub-units of the rotor.

In an embodiment, the system further comprises a temperature sensor, and wherein the temperature control system adjusts the temperature of the sample region in response to an output of the temperature sensor.

In an embodiment, the temperature sensor comprises a thermocouple.

In an embodiment, the temperature sensor comprises an optical sensor.

In an embodiment, the temperature sensor comprises an infrared sensor.

In an embodiment, the temperature sensor is one of a plurality of temperature sensors.

In an embodiment, at least two of the plurality of temperature sensors are in communication with each other.

In an embodiment, the temperature control system comprises an open-loop temperature system.

In an embodiment, the temperature control system comprises a closed-loop temperature feedback system.

In an embodiment, the system further comprises a sample chamber at the sample region and wherein the temperature control system is configured to adjust a temperature of the sample chamber.

In an embodiment, the temperature control system is positioned at the sample region.

In an embodiment, the temperature control system comprises a thermoelectric or Peltier device.

In an embodiment, the rotor comprises one or more sub-units configured to be stacked in a vertical direction of extension along the axis of rotation and wherein one or more of the one or more stacked sub-units comprises a power source.

In an embodiment, the power source comprises at least one battery.

In an embodiment, the at least one battery is positioned on a first sub-unit and supplies power to a device on a second sub-unit.

In an embodiment, each of the one or more sub-units comprises a power source.

In an embodiment, the power source comprises a recharging mechanism constructed and arranged to convert the rotational energy of the rotor into electrical current.

In an embodiment, the recharging mechanism comprises at least one voltaic cell.

In an embodiment, the at least one voltaic cell comprises two electrodes separated by an electrolyte solution.

In an embodiment, the current is driven by an electrolyte concentration difference in the electrolyte solution that is induced by centrifugation.

In an embodiment, the at least one voltaic cell comprises a concentration cell.

In an embodiment, the at least one voltaic cell is driven by a radio-isotopic decay.

In an embodiment, the at least one voltaic cell comprises a beta-voltaic cell.

In an embodiment, the system further comprises a recharging mechanism that recharges the battery, the recharging mechanism converting rotational energy from the rotation of the rotor into electrical current.

In an embodiment, the recharging mechanism comprises an electric generator comprising a planetary gear configuration constructed and arranged to facilitate relative internal rotation in a rotor-stator pairing.

In an embodiment, an integrated rotor system, comprises: a rotor comprising at least one rotor cavity, the rotor being constructed and arranged to rotate about an axis of rotation; and an interrogation cell positioned in the at least one rotor cavity. The interrogation cell comprises: a source of electromagnetic radiation at a first position of the interrogation cell, the source of electromagnetic radiation configured to emit electromagnetic radiation at one or more wavelengths; a sample region; and a detector at a second position of the interrogation cell, the detector configured to receive electromagnetic radiation that traverses at least a portion of the sample region.

In an embodiment, the at least one rotor cavity comprises a plurality of rotor cavities.

In an embodiment, the system further comprises a plurality of interrogation cells, each interrogation cell corresponding to one of the plurality of rotor cavities.

In an embodiment, the rotor comprises a central opening.

In an embodiment, the central opening is aligned with the axis of rotation.

In an embodiment, the rotor is constructed and arranged to mate with a spindle.

In an embodiment, the spindle is aligned with the axis of rotation.

In an embodiment, the spindle comprises a spindle of a centrifuge.

In an embodiment, the rotor is oriented symmetrically about the axis of rotation.

In an embodiment, the interrogation cell comprises one or more sub-units configured to be stacked in a vertical direction of extension along the axis of rotation.

In an embodiment, neighboring ones of the one or more stacked sub-units are coupled to each other at a threaded interface.

In an embodiment, one or more of the one or more stacked sub-units are coupled with a bolt-through configuration.

In an embodiment, a gasket is positioned between one or more of the one or more stacked sub-units.

In an embodiment, the gasket is constructed and arranged to form a sealed region.

In an embodiment, the sealed region is sealed from an ambient region external to the sealed region.

In an embodiment, one or more of the one or more sub-units are coupled to each other with a tongue-in-groove structure.

In an embodiment, one or more of the one or more stacked sub-units comprise high-strength materials.

In an embodiment, the high-strength material comprises titanium.

In an embodiment, the high-strength material comprises an alloy material.

In an embodiment, the high-strength material comprises a composite material.

In an embodiment, the high-strength material comprises a material including a carbon fiber material.

In an embodiment, the sample region comprises a sector shape.

In an embodiment, the sample region comprises a sample chamber.

In an embodiment, the sample chamber comprises a removable liner.

In an embodiment, the first position and the second position are at the sample chamber and wherein the sample chamber comprises the source of electromagnetic radiation and the detector.

In an embodiment, the sample region comprises an open top and an open bottom.

In an embodiment, the sample region is coupled to one or more neighboring sub-units.

In an embodiment, a portion of the electromagnetic radiation emitted from the source of electromagnetic radiation is incident on the detector.

In an embodiment, the electromagnetic radiation is directed toward the sample region.

In an embodiment, the detector is arranged to detect electromagnetic radiation that traverses a portion of the sample region.

In an embodiment, the sample region, the source of electromagnetic radiation, and the detector are arranged along an axis that is parallel to the axis of rotation.

In an embodiment, the source of electromagnetic radiation is positioned at a first of the one or more stacked sub-units.

In an embodiment, the sample region is positioned at a second of the one or more stacked sub-units.

In an embodiment, the detector is positioned at a third of the one or more stacked sub-units.

In an embodiment, the system further comprises an alignment mechanism that aligns the first, second and third of the one or more stacked sub-units so that the source of electromagnetic radiation, the sample region and the detector being aligned in a vertical direction along an axis of interrogation that is parallel to the axis of rotation.

In an embodiment, a portion of the electromagnetic radiation emitted from the source of electromagnetic radiation traverses a portion of the sample chamber.

In an embodiment, one or more wavelengths of the electromagnetic radiation is incident at the sample region.

In an embodiment, electromagnetic radiation propagates through the sample region to the detector.

In an embodiment, electromagnetic radiation triggers a reaction of material at the sample region.

In an embodiment, the detector collects electromagnetic radiation emitted from the sample region and the electromagnetic radiation emitted from the sample region comprises one or more wavelengths that are different than the one or more wavelengths emitted from the source of electromagnetic radiation.

In an embodiment, the wavelengths of the electromagnetic radiation emitted from the sample region are greater than the one or more wavelengths emitted from the source of electromagnetic radiation.

In an embodiment, the wavelengths of the electromagnetic radiation emitted from the sample region are less than the one or more wavelengths emitted from the source of electromagnetic radiation.

In an embodiment, the electromagnetic radiation triggers a photophysical interaction.

In an embodiment, the photophysical interaction comprises excitation of molecules present in the sample region to energy levels above a ground state of the sample molecules.

In an embodiment, the excitation of the energy levels consists of at least one of excitation of electronic energy levels, excitation of vibrational energy levels or excitation of rotational energy levels.

In an embodiment, the source of electromagnetic radiation is one of a plurality of sources of electromagnetic radiation that is optically coupled with the detector.

In an embodiment, the source of electromagnetic radiation is coupled to a circuit board.

In an embodiment, the interrogation cell comprises one or more sub-units configured to be stacked in a vertical direction of extension along the axis of rotation and wherein a topmost sub-unit of the one or more sub-units comprises a source of electromagnetic radiation coupled to a circuit board.

In an embodiment, the interrogation cell comprises one or more sub-units configured to be stacked in a vertical direction of extension along the axis of rotation and wherein a topmost sub-unit of the one or more sub-units comprises a plurality of sources of electromagnetic radiation, wherein the sample region comprises a plurality of sample regions and wherein the detector comprises a plurality of detectors, each source of electromagnetic radiation being optically coupled to a corresponding detector through an optical path that includes a corresponding sample region.

In an embodiment, the source of electromagnetic radiation is a single light emitting diode (LED).

In an embodiment, the source of electromagnetic radiation comprises an array of light emitting diodes (LEDs).

In an embodiment, the source of electromagnetic radiation comprises a laser diode.

In an embodiment, the source of electromagnetic radiation comprises and array of laser diodes.

In an embodiment, the detector comprises an array detector.

In an embodiment, the detector comprises a point detector.

In an embodiment, the detector comprises a CCD array.

In an embodiment, the detector comprises a photodiode.

In an embodiment, the detector periodically samples incident electromagnetic radiation.

In an embodiment, the system further comprises a storage that stores information collected by the detector.

In an embodiment, the system further comprises a transmitter that transmits the stored information stored by the storage to a receiver.

In an embodiment, the transmitter transmits the stored information as the rotor is rotating about the axis of rotation.

In an embodiment, the system transmits the stored information when the rotor is stationary.

In an embodiment, the system is configured to measure light absorbance of the sample region based on the stored information collected by the detector.

In an embodiment, the system is configured to provide spectroscopic information of the sample region based on the stored information collected by the detector.

In an embodiment, the system is configured to provide hyperspectral image data of the sample region based on the stored information collected by the detector.

In an embodiment, the system is configured to provide Schlieren images of the sample region based on the stored information collected by the detector.

In an embodiment, the system is configured to provide fluorescence images of the sample region based on the stored information collected by the detector.

In an embodiment, the system is configured to provide quantitative fluorescence emission data of the sample region based on the stored information collected by the detector.

In an embodiment, the quantitative fluorescence emission data comprises a spatial arrangement of the sample region.

In an embodiment, the system further comprises a Fabry-Perot interferometer optically coupled with the source of electromagnetic radiation and the detector.

In an embodiment, the system further comprises one or more optical filters positioned between the source of electromagnetic radiation and the detector.

In an embodiment, the system further comprises one or more optical lenses positioned between the source of electromagnetic radiation and the detector.

In an embodiment, the system further comprises one or more mirrors positioned between the source of electromagnetic radiation and the detector.

In an embodiment, the system further comprises one or more optical diffusers positioned between the source of electromagnetic radiation and the detector.

In an embodiment, the system further comprises one or more optical collimators positioned between the source of electromagnetic radiation and the detector.

In an embodiment, one or more of the one or more optical collimators comprises at least one self-collimating photonic crystal.

In an embodiment, one or more of the one or more optical collimators comprises at least one micro-Fresnel lens.

In an embodiment, the system further comprises one or more optical lenses positioned between the source of electromagnetic radiation and the detector wherein the one or more optical lenses are positioned relative to the sample region and the detector such that the detector detects an image of a plane at the sample region.

In an embodiment, the one or more lenses are positioned below the sample region.

In an embodiment, the system further comprises an elongated edge constructed and arranged to block a portion of the light incident on the detector.

In an embodiment, the system further comprises an iris constructed and arranged to block a portion of the light incident on the detector.

In an embodiment, the system further comprises a beamsplitter system constructed and arranged to direct electromagnetic radiation from the source of electromagnetic radiation to a plurality of locations.

In an embodiment, the beamsplitter system comprises at least one mirror.

In an embodiment, the beamsplitter system comprises at least one filter.

In an embodiment, the beamsplitter system comprises at least one lens.

In an embodiment, the beamsplitter system is constructed and arranged such that electromagnetic radiation emitted from a first sub-unit of the interrogation cell is optically coupled to a detector on a second sub-unit of the interrogation cell different than the first sub-unit.

In an embodiment, the interrogation cell comprises one or more sub-units configured to be stacked in a vertical direction of extension along the axis of rotation and wherein one or more of the one or more stacked sub-units are electrically connected with one or more connectors.

In an embodiment, the one or more connectors are configured to transfer information.

In an embodiment, the one or more connectors are configured to transfer power.

In an embodiment, one or more of the one or more stacked sub-units are electrically connected with USB ports.

In an embodiment, one or more of the one or more stacked sub-units are electrically connected with micro-USB ports.

In an embodiment, one or more of the one or more stacked sub-units are electrically connected with VGA ports.

In an embodiment, one or more of the one or more stacked sub-units are electrically connected with D-type connectors.

In an embodiment, the system further comprises a transmitter configured to transmit information from the detector.

In an embodiment, the transmitter is configured to transmit the information in an encrypted format.

In an embodiment, the transmitter is configured to transmit the information to a cloud-based storage system.

In an embodiment, the transmitter is configured to transmit information wirelessly.

In an embodiment, the transmitter comprises an antenna.

In an embodiment, the transmitter is configured to transmit the information optically.

In an embodiment, the transmitter comprises a cable connection.

In an embodiment, the cable connection comprises an electrical connection.

In an embodiment, the cable connection comprises fiber optics.

In an embodiment, the cable connection comprises connection to a circuit board.

In an embodiment, the cable connection comprises connection to an on-board memory.

In an embodiment, the on-board memory comprises a device selected from the group consisting of: a random access memory (RAM) device, a read-only memory (ROM) device, a solid-state memory (SSD) device, an SD memory card, or a micro-SD memory card.

In an embodiment, the transmitter is positioned at a bottom portion of the interrogation cell.

In an embodiment, the rotor comprises a central opening aligned with the axis of rotation.

In an embodiment, the transmitter is constructed and arranged to extend through the central opening.

In an embodiment, the transmitter extends from the bottom of the rotor to the top of the rotor.

In an embodiment, the transmitter comprises one of a plurality of transmitters.

In an embodiment, the transmitter comprises a battery.

In an embodiment, the system further comprises a recharging mechanism that recharges the battery, the recharging mechanism converting rotational energy from the rotation of the rotor into electrical current.

In an embodiment, the recharging mechanism comprises an electric generator comprising a planetary gear configuration constructed and arranged to facilitate relative internal rotation in a rotor-stator pairing.

In an embodiment, the system further comprises an on-board memory at the interrogation cell.

In an embodiment, the on-board memory comprises a device selected from the group consisting of: a random access memory (RAM) device, a read-only memory (ROM) device, a solid-state memory (SSD) device, an SD memory card, or a micro-SD memory card.

In an embodiment, the system further comprises a temperature control system configured to modify a temperature of the sample region.

In an embodiment, the temperature control system is configured to maintain the temperature of the sample region.

In an embodiment, maintaining the temperature comprises heating the sample region.

In an embodiment, maintaining the temperature comprises cooling the sample region.

In an embodiment, the temperature control system circulates thermally conductive material at the sample region.

In an embodiment, the temperature control system comprises a pumping system to circulate the thermally conductive material.

In an embodiment, the temperature control system heats the sample region.

In an embodiment, the temperature control system cools the sample region.

In an embodiment, the temperature control system circulates thermally conductive material through channels at one or more sub-units of the interrogation cell.

In an embodiment, the system further comprises a temperature sensor, and wherein the temperature control system adjusts the temperature of the sample region in response to an output of the temperature sensor.

In an embodiment, the temperature sensor comprises a thermocouple.

In an embodiment, the temperature sensor comprises an optical sensor.

In an embodiment, the temperature sensor comprises an infrared sensor.

In an embodiment, the temperature sensor is one of a plurality of temperature sensors.

In an embodiment, at least two of the plurality of temperature sensors are in communication with each other.

In an embodiment, the temperature control system comprises an open-loop temperature system.

In an embodiment, the temperature control system comprises a closed-loop temperature feedback system.

In an embodiment, the system further comprises a sample chamber at the sample region and wherein the temperature control system is configured to adjust a temperature of the sample chamber.

In an embodiment, the temperature control system is positioned at the sample region.

In an embodiment, the temperature control system comprises a thermoelectric or Peltier device.

In an embodiment, the interrogation cell comprises one or more sub-units configured to be stacked in a vertical direction of extension along the axis of rotation and wherein one or more of the one or more stacked sub-units comprises a power source.

In an embodiment, the power source comprises at least one battery.

In an embodiment, the at least one battery is positioned on a first sub-unit and supplies power to a device on a second sub-unit.

In an embodiment, each of the one or more sub-units comprises a power source.

In an embodiment, the power source comprises a recharging mechanism constructed and arranged to convert the rotational energy of the rotor into electrical current.

In an embodiment, the recharging mechanism comprises at least one voltaic cell.

In an embodiment, the at least one voltaic cell comprises two electrodes separated by an electrolyte solution.

In an embodiment, the current is driven by an electrolyte concentration difference in the electrolyte solution that is induced by centrifugation.

In an embodiment, the at least one voltaic cell comprises a concentration cell.

In an embodiment, the at least one voltaic cell is driven by a radio-isotopic decay.

In an embodiment, the at least one voltaic cell comprises a beta-voltaic cell.

In an embodiment, the system further comprises a recharging mechanism that recharges the battery, the recharging mechanism converting rotational energy from the rotation of the rotor into electrical current.

In an embodiment, the recharging mechanism comprises an electric generator comprising a planetary gear configuration constructed and arranged to facilitate relative internal rotation in a rotor-stator pairing.

In an embodiment, an interrogation cell, comprises; a source of electromagnetic radiation at a first position, the source of electromagnetic radiation configured to emit electromagnetic radiation at one or more wavelengths; a sample region; and a detector at a second position, the detector configured to receive electromagnetic radiation that traverses at least a portion of the sample region, wherein the interrogation cell is dimensioned for positioning in a rotor cavity of a centrifuge rotor.

In an embodiment, the interrogation cell comprises one or more sub-units configured to be stacked in a vertical direction of extension along the axis of rotation.

In an embodiment, neighboring ones of the one or more stacked sub-units are coupled to each other at a threaded interface.

In an embodiment, one or more of the one or more stacked sub-units are coupled with a bolt-through configuration.

In an embodiment, a gasket is positioned between one or more of the one or more stacked sub-units.

In an embodiment, the gasket is constructed and arranged to form a sealed region.

In an embodiment, the sealed region is sealed from an ambient region external to the sealed region.

In an embodiment, one or more of the one or more sub-units are coupled to each other with a tongue-in-groove structure.

In an embodiment, one or more of the one or more stacked sub-units comprise high-strength materials.

In an embodiment, the high-strength material comprises titanium.

In an embodiment, the high-strength material comprises an alloy material.

In an embodiment, the high-strength material comprises a composite material.

In an embodiment, the high-strength material comprises a material including a carbon fiber material.

In an embodiment, the sample region comprises a sector shape.

In an embodiment, the sample region comprises a sample chamber.

In an embodiment, the sample chamber comprises a removable liner.

In an embodiment, the first position and the second position are at the sample chamber and wherein the sample chamber comprises the source of electromagnetic radiation and the detector.

In an embodiment, the sample region comprises an open top and an open bottom.

In an embodiment, the sample region is coupled to one or more neighboring sub-units.

In an embodiment, a portion of the electromagnetic radiation emitted from the source of electromagnetic radiation is incident on the detector.

In an embodiment, the electromagnetic radiation is directed toward the sample region.

In an embodiment, the detector is arranged to detect electromagnetic radiation that traverses a portion of the sample region.

In an embodiment, the sample region, the source of electromagnetic radiation, and the detector are arranged along an axis that is parallel to the axis of rotation.

In an embodiment, the source of electromagnetic radiation is positioned at a first of the one or more stacked sub-units.

In an embodiment, the sample region is positioned at a second of the one or more stacked sub-units.

In an embodiment, the detector is positioned at a third of the one or more stacked sub-units.

In an embodiment, the system further comprises an alignment mechanism that aligns the first, second and third of the one or more stacked sub-units so that the source of electromagnetic radiation, the sample region and the detector being aligned in a vertical direction along an axis of interrogation that is parallel to the axis of rotation.

In an embodiment, a portion of the electromagnetic radiation emitted from the source of electromagnetic radiation traverses a portion of the sample chamber.

In an embodiment, one or more wavelengths of the electromagnetic radiation is incident at the sample region.

In an embodiment, electromagnetic radiation propagates through the sample region to the detector.

In an embodiment, electromagnetic radiation triggers a reaction of material at the sample region.

In an embodiment, the detector collects electromagnetic radiation emitted from the sample region and the electromagnetic radiation emitted from the sample region comprises one or more wavelengths that are different than the one or more wavelengths emitted from the source of electromagnetic radiation.

In an embodiment, the wavelengths of the electromagnetic radiation emitted from the sample region are greater than the one or more wavelengths emitted from the source of electromagnetic radiation.

In an embodiment, the wavelengths of the electromagnetic radiation emitted from the sample region are less than the one or more wavelengths emitted from the source of electromagnetic radiation.

In an embodiment, the electromagnetic radiation triggers a photophysical interaction.

In an embodiment, the photophysical interaction comprises excitation of molecules present in the sample region to energy levels above a ground state of the sample molecules.

In an embodiment, the excitation of the energy levels consists of at least one of excitation of electronic energy levels, excitation of vibrational energy levels or excitation of rotational energy levels.

In an embodiment, the source of electromagnetic radiation is one of a plurality of sources of electromagnetic radiation that is optically coupled with the detector.

In an embodiment, the source of electromagnetic radiation is coupled to a circuit board.

In an embodiment, the interrogation cell comprises one or more sub-units configured to be stacked in a vertical direction of extension along the axis of rotation and wherein a topmost sub-unit of the one or more sub-units comprises a source of electromagnetic radiation coupled to a circuit board.

In an embodiment, the interrogation cell comprises one or more sub-units configured to be stacked in a vertical direction of extension along the axis of rotation and wherein a topmost sub-unit of the one or more sub-units comprises a plurality of sources of electromagnetic radiation, wherein the sample region comprises a plurality of sample regions and wherein the detector comprises a plurality of detectors, each source of electromagnetic radiation being optically coupled to a corresponding detector through an optical path that includes a corresponding sample region.

In an embodiment, the source of electromagnetic radiation is a single light emitting diode (LED).

In an embodiment, the source of electromagnetic radiation comprises an array of light emitting diodes (LEDs).

In an embodiment, the source of electromagnetic radiation comprises a laser diode.

In an embodiment, the source of electromagnetic radiation comprises and array of laser diodes.

In an embodiment, the detector comprises an array detector.

In an embodiment, the detector comprises a point detector.

In an embodiment, the detector comprises a CCD array.

In an embodiment, the detector comprises a photodiode.

In an embodiment, the detector periodically samples incident electromagnetic radiation.

In an embodiment, the system further comprises a storage that stores information collected by the detector.

In an embodiment, the system further comprises a transmitter that transmits the stored information stored by the storage to a receiver.

In an embodiment, the transmitter transmits the stored information as the interrogation cell is rotating about the axis of rotation of the rotor.

In an embodiment, the system transmits the stored information when the interrogation cell is stationary.

In an embodiment, the system is configured to measure light absorbance of the sample region based on the stored information collected by the detector.

In an embodiment, the system is configured to provide spectroscopic information of the sample region based on the stored information collected by the detector.

In an embodiment, the system is configured to provide hyperspectral image data of the sample region based on the stored information collected by the detector.

In an embodiment, the system is configured to provide Schlieren images of the sample region based on the stored information collected by the detector.

In an embodiment, the system is configured to provide fluorescence images of the sample region based on the stored information collected by the detector.

In an embodiment, the system is configured to provide quantitative fluorescence emission data of the sample region based on the stored information collected by the detector.

In an embodiment, the quantitative fluorescence emission data comprises a spatial arrangement of the sample region.

In an embodiment, the system further comprises a Fabry-Perot interferometer optically coupled with the source of electromagnetic radiation and the detector.

In an embodiment, the system further comprises one or more optical filters positioned between the source of electromagnetic radiation and the detector.

In an embodiment, the system further comprises one or more optical lenses positioned between the source of electromagnetic radiation and the detector.

In an embodiment, the system further comprises one or more mirrors positioned between the source of electromagnetic radiation and the detector.

In an embodiment, the system further comprises one or more optical diffusers positioned between the source of electromagnetic radiation and the detector.

In an embodiment, the system further comprises one or more optical collimators positioned between the source of electromagnetic radiation and the detector.

In an embodiment, one or more of the one or more optical collimators comprises at least one self-collimating photonic crystal.

In an embodiment, one or more of the one or more optical collimators comprises at least one micro-Fresnel lens.

In an embodiment, the system further comprises one or more optical lenses positioned between the source of electromagnetic radiation and the detector wherein the one or more optical lenses are positioned relative to the sample region and the detector such that the detector detects an image of a plane at the sample region.

In an embodiment, the one or more lenses are positioned below the sample region.

In an embodiment, the system further comprises an elongated edge constructed and arranged to block a portion of the light incident on the detector.

In an embodiment, the system further comprises an iris constructed and arranged to block a portion of the light incident on the detector.

In an embodiment, the system further comprises a beamsplitter system constructed and arranged to direct electromagnetic radiation from the source of electromagnetic radiation to a plurality of locations.

In an embodiment, the beamsplitter system comprises at least one mirror.

In an embodiment, the beamsplitter system comprises at least one filter.

In an embodiment, the beamsplitter system comprises at least one lens.

In an embodiment, the beamsplitter system is constructed and arranged such that electromagnetic radiation emitted from a first sub-unit of the interrogation cell is optically coupled to a detector on a second sub-unit of the interrogation cell different than the first sub-unit.

In an embodiment, the interrogation cell comprises one or more sub-units configured to be stacked in a vertical direction of extension along the axis of rotation and wherein one or more of the one or more stacked sub-units are electrically connected with one or more connectors.

In an embodiment, the one or more connectors are configured to transfer information.

In an embodiment, the one or more connectors are configured to transfer power.

In an embodiment, one or more of the one or more stacked sub-units are electrically connected with USB ports.

In an embodiment, one or more of the one or more stacked sub-units are electrically connected with micro-USB ports.

In an embodiment, one or more of the one or more stacked sub-units are electrically connected with VGA ports.

In an embodiment, one or more of the one or more stacked sub-units are electrically connected with D-type connectors.

In an embodiment, the system further comprises a transmitter configured to transmit information from the detector.

In an embodiment, the transmitter is configured to transmit the information in an encrypted format.

In an embodiment, the transmitter is configured to transmit the information to a cloud-based storage system.

In an embodiment, the transmitter is configured to transmit information wirelessly.

In an embodiment, the transmitter comprises an antenna.

In an embodiment, the transmitter is configured to transmit the information optically.

In an embodiment, the transmitter comprises a cable connection.

In an embodiment, the cable connection comprises an electrical connection.

In an embodiment, the cable connection comprises fiber optics.

In an embodiment, the cable connection comprises connection to a circuit board.

In an embodiment, the cable connection comprises connection to an on-board memory.

In an embodiment, the on-board memory comprises a device selected from the group consisting of: a random access memory (RAM) device, a read-only memory (ROM) device, a solid-state memory (SSD) device, an SD memory card, or a micro-SD memory card.

In an embodiment, the transmitter is positioned at a bottom portion of the interrogation cell.

In an embodiment, the transmitter comprises one of a plurality of transmitters.

In an embodiment, the transmitter comprises a battery.

In an embodiment, the system further comprises an on-board memory at the interrogation cell.

In an embodiment, the on-board memory comprises a device selected from the group consisting of: a random access memory (RAM) device, a read-only memory (ROM) device, a solid-state memory (SSD) device, an SD memory card, or a micro-SD memory card.

In an embodiment, the system further comprises a temperature control system configured to modify a temperature of the sample region.

In an embodiment, the temperature control system is configured to maintain the temperature of the sample region.

In an embodiment, maintaining the temperature comprises heating the sample region.

In an embodiment, maintaining the temperature comprises cooling the sample region.

In an embodiment, the temperature control system circulates thermally conductive material at the sample region.

In an embodiment, the temperature control system comprises a pumping system to circulate the thermally conductive material.

In an embodiment, the temperature control system heats the sample region.

In an embodiment, the temperature control system cools the sample region.

In an embodiment, the temperature control system circulates thermally conductive material through channels at one or more sub-units of the interrogation cell.

In an embodiment, the system further comprises a temperature sensor, and wherein the temperature control system adjusts the temperature of the sample region in response to an output of the temperature sensor.

In an embodiment, the temperature sensor comprises a thermocouple.

In an embodiment, the temperature sensor comprises an optical sensor.

In an embodiment, the temperature sensor comprises an infrared sensor.

In an embodiment, the temperature sensor is one of a plurality of temperature sensors.

In an embodiment, at least two of the plurality of temperature sensors are in communication with each other.

In an embodiment, the temperature control system comprises an open-loop temperature system.

In an embodiment, the temperature control system comprises a closed-loop temperature feedback system.

In an embodiment, the system further comprises a sample chamber at the sample region and wherein the temperature control system is configured to adjust a temperature of the sample chamber.

In an embodiment, the temperature control system is positioned at the sample region.

In an embodiment, the temperature control system comprises a thermoelectric or Peltier device.

In an embodiment, the interrogation cell comprises one or more sub-units configured to be stacked in a vertical direction of extension along the axis of rotation and wherein one or more of the one or more stacked sub-units comprises a power source.

In an embodiment, the power source comprises at least one battery.

In an embodiment, the at least one battery is positioned on a first sub-unit and supplies power to a device on a second sub-unit.

In an embodiment, each of the one or more sub-units comprises a power source.

In an embodiment, the power source comprises a recharging mechanism constructed and arranged to convert the rotational energy of the interrogation cell into electrical current.

In an embodiment, the recharging mechanism comprises at least one voltaic cell.

In an embodiment, the at least one voltaic cell comprises two electrodes separated by an electrolyte solution.

In an embodiment, the current is driven by an electrolyte concentration difference in the electrolyte solution that is induced by centrifugation.

In an embodiment, the at least one voltaic cell comprises a concentration cell.

In an embodiment, the at least one voltaic cell is driven by a radio-isotopic decay.

In an embodiment, the at least one voltaic cell comprises a beta-voltaic cell.

In an embodiment, the system further comprises a recharging mechanism that recharges the battery, the recharging mechanism converting rotational energy from the rotation of the interrogation cell into electrical current.

In an aspect, a method comprises: providing a rotor constructed and arranged to rotate about an axis of rotation; providing a source of electromagnetic radiation at a first position of the rotor, the source of electromagnetic radiation configured to emit electromagnetic radiation at one or more wavelengths; providing a sample region; and providing a detector at a second position of the rotor, the detector constructed and arranged to receive electromagnetic radiation that traverses at least a portion of the sample region.

In an embodiment, the method further comprises mounting the rotor system at a centrifuge.

In an embodiment, a method comprises: providing a rotor comprising at least one rotor cavity, the rotor being constructed and arranged to rotate about an axis of rotation; and providing an interrogation cell positioned in the at least one rotor cavity, comprising: providing a source of electromagnetic radiation at a first position of the interrogation cell, the source of electromagnetic radiation configured to emit electromagnetic radiation at one or more wavelengths; providing a sample region; and providing a detector at a second position of the interrogation cell, the detector configured to receive electromagnetic radiation that traverses at least a portion of the sample region.

In an embodiment, the method further comprises mounting the rotor system at a centrifuge.

In an embodiment, a method of providing an interrogation cell, comprises: providing a source of electromagnetic radiation, the source of electromagnetic radiation configured to emit electromagnetic radiation at one or more wavelengths; providing a sample region; and providing a detector at a second position, the detector configured to receive electromagnetic radiation that traverses at least a portion of the sample region, wherein the interrogation cell is dimensioned for positioning in a rotor cavity of a centrifuge rotor.

In an embodiment, the method further comprises mounting the interrogation cell at a centrifuge rotor.

In an embodiment, the method further comprises mounting the centrifuge rotor including the interrogation cell at a centrifuge.

### BRIEF DESCRIPTION OF THE DRAWINGS

The foregoing and other objects, features and advantages of embodiments of the present inventive concepts will be apparent from the more particular description of embodiments, as illustrated in the accompanying drawings in which like reference characters refer to the same elements throughout the different views. The drawings are not necessarily to scale, emphasis instead being placed upon illustrating the principles of the embodiments.
FIG. 1 is a perspective view of the rotor body depicting a possible embodiment of the external form.
FIG. 2 is a perspective view having a cutaway section to illustrate an embodiment of possible internal structure in the device.
FIG. 3 is a diagram of a section of internal components illustrating a possible functional set-up and connectivity in a measurement system.
FIG. 4 is a side elevation cutaway section diagram illustrating an embodiment wherein the functional components are located on modular units of a rotor that can be assembled/disassembled to form a functional rotor device.
FIG. 5 is a perspective view having a cutaway section to illustrate an embodiment wherein the functional components are located on one or more independent cell units and whereby the functional cells are then affixed inside a rotor.
FIG. 6 is a top-down (overhead) view of an optical cavity illustrating an embodiment where components and interfaces are positioned.
FIG. 7 is a cutaway (section) view of an embodiment of an optical cavity, including the orientation of fluid chamber, light source and detector, and other components within the cavity.
FIG. 8A is a perspective sectional view of an embodiment of a rotor system, in accordance with aspects of the present inventive concepts.
FIG. 8B is an exploded perspective view of an embodiment with four subunits, in accordance with aspects of the present inventive concepts.
FIG. 8C is an exploded perspective view of an embodiment with four subunits, in accordance with aspects of the present inventive concepts.
FIG. 8D is an exploded perspective view of an embodiment with four subunits, in accordance with aspects of the present inventive concepts.
FIG. 8E is a perspective view of an embodiment with four subunits assembled, in accordance with aspects of the present inventive concepts.
FIG. 8F is a perspective view of an embodiment of a subunit comprising a sealing gasket, in accordance with aspects of the present inventive concepts.
FIG. 8G is a perspective view of an embodiment of a subunit including a functional element, in accordance with aspects of the present inventive concepts.

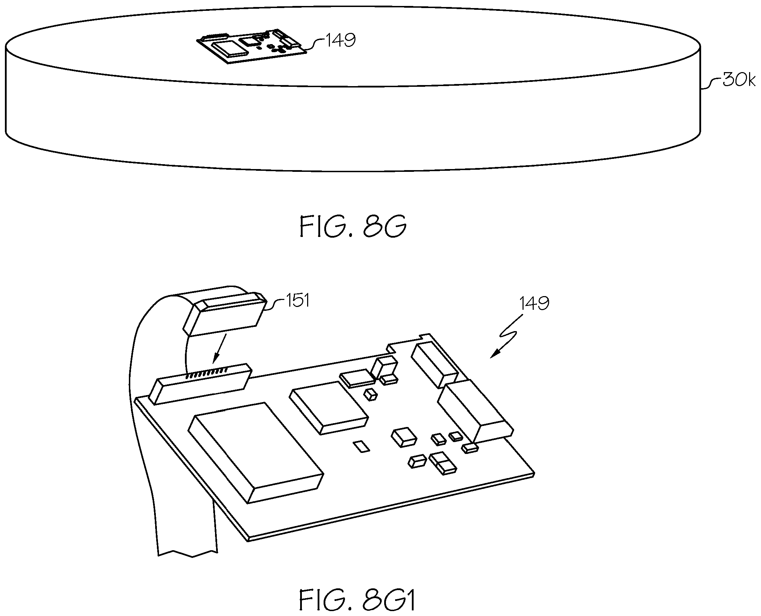

FIG. 8G1 is a close-up perspective view of an embodiment of a functional element and an associated connector, in accordance with aspects of the present inventive concepts.

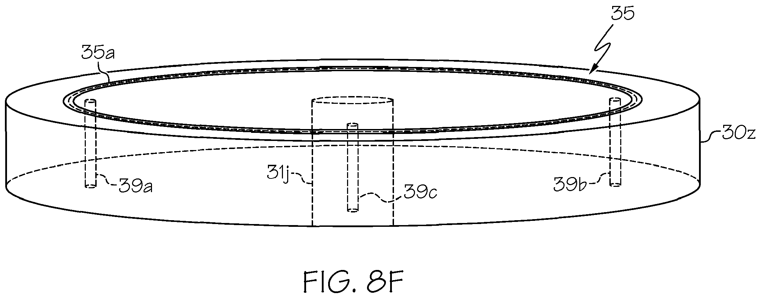

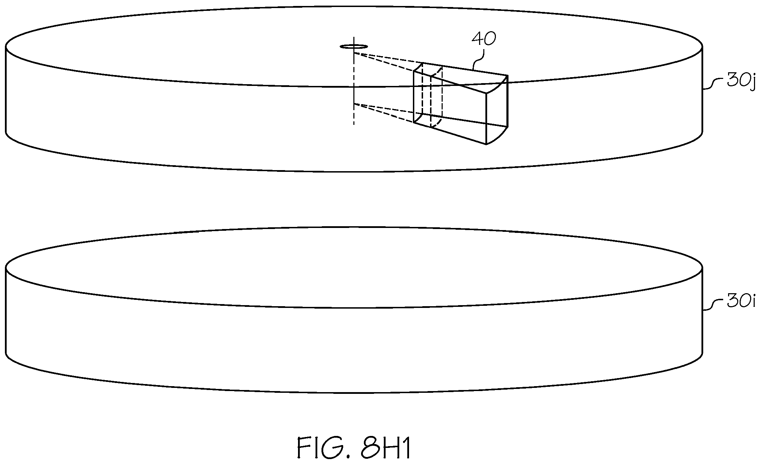

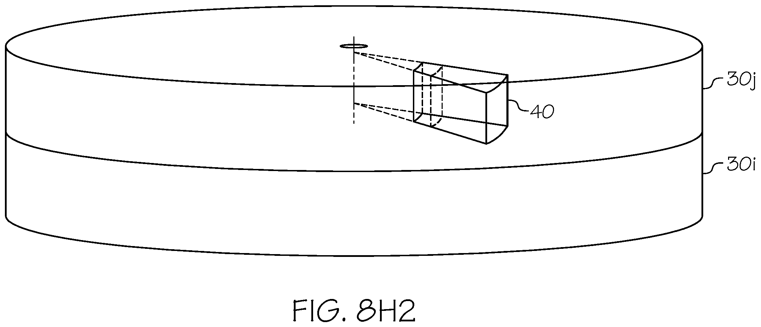

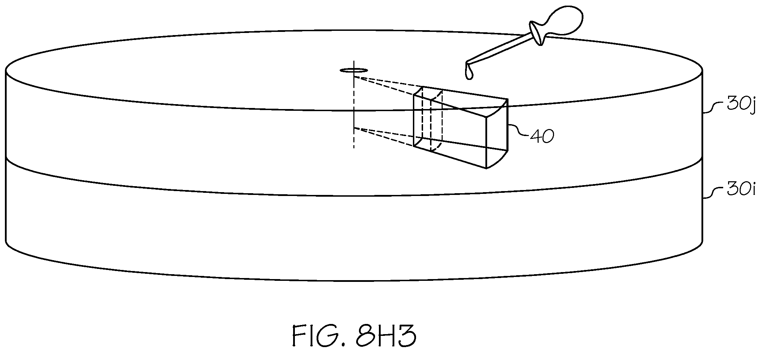

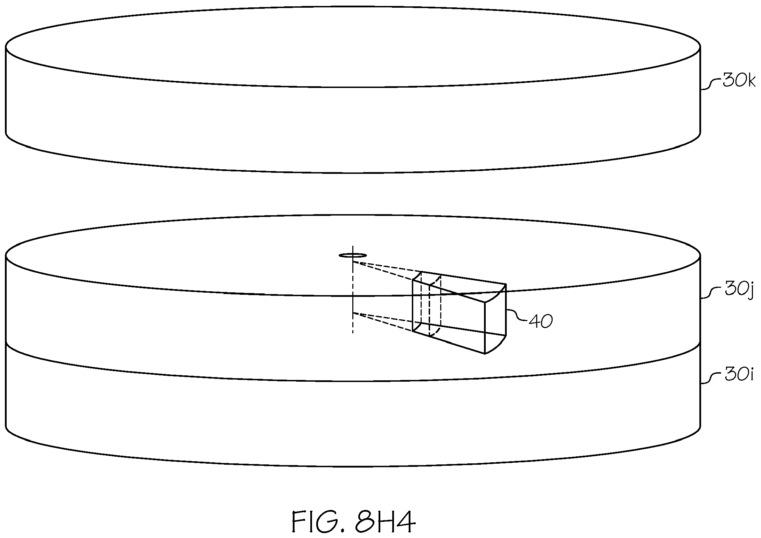

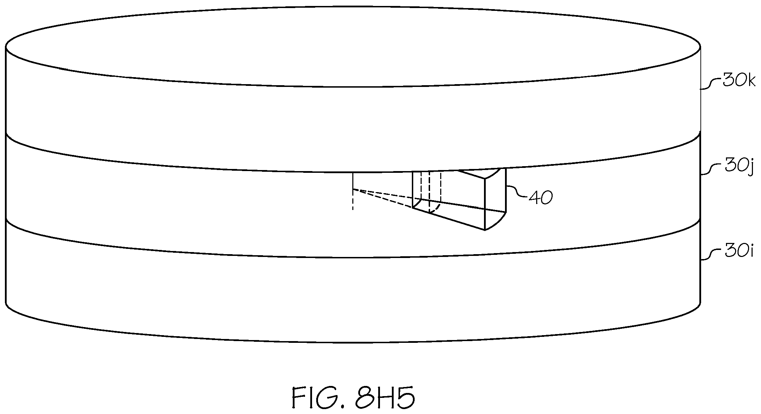

FIG. 8H1 is a perspective view of an embodiment of the two subunits of FIG. 8F1, in accordance with aspects of the present inventive concepts.
FIG. 8H2 is a perspective view of an embodiment of the two subunits of FIG. 8F1 coupled with an air-tight and liquid-tight junction, in accordance with aspects of the present inventive concepts.
FIG. 8H3 is a perspective view of a sample being added to the sample region, in accordance with aspects of the present inventive concepts.
FIG. 8H4 is a perspective view of an embodiment of a subunit being added above the sample region, in accordance with aspect of the present inventive concepts.
FIG. 8H5 is a perspective view of an embodiment of three subunits coupled together, in accordance with aspects of the present inventive concepts.

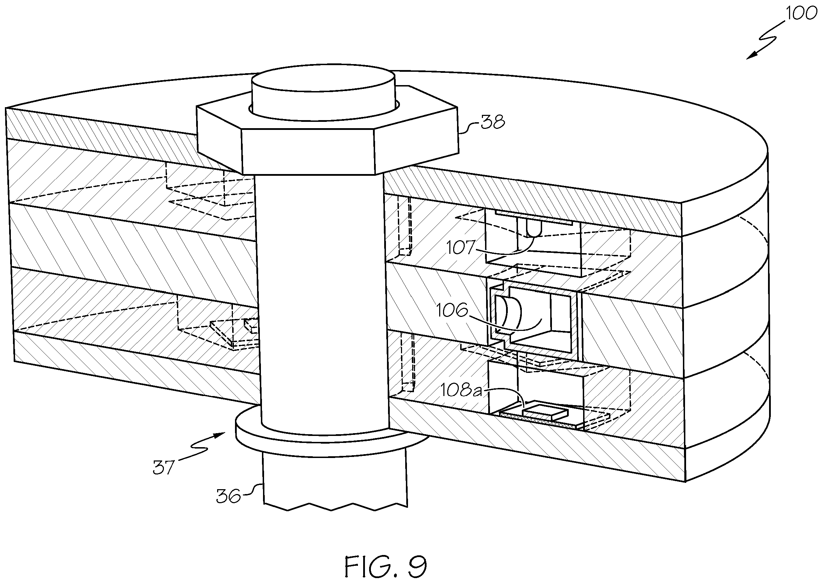

FIG. 9 is a perspective sectional view of an embodiment of a rotor system, in accordance with aspects of the present inventive concepts.

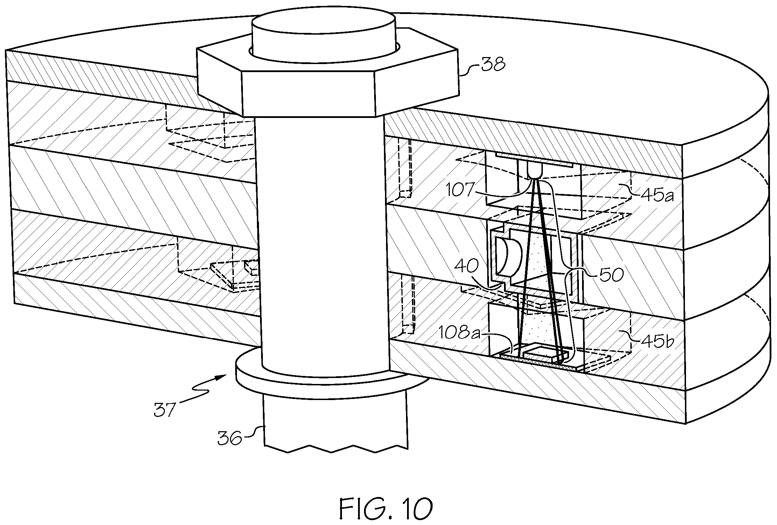

FIG. 10 is a perspective sectional view of an embodiment of a rotor system, in accordance with aspects of the present inventive concepts.

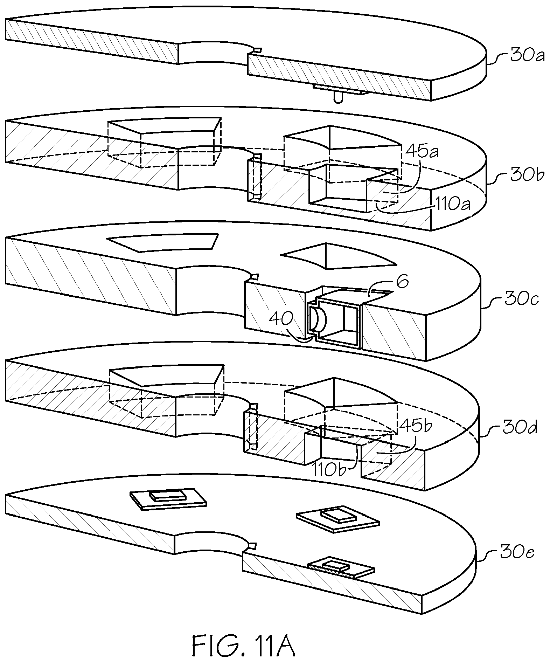

FIG. 11A is an exploded perspective view of an embodiment of a rotor system, in accordance with aspects of the present inventive concepts.

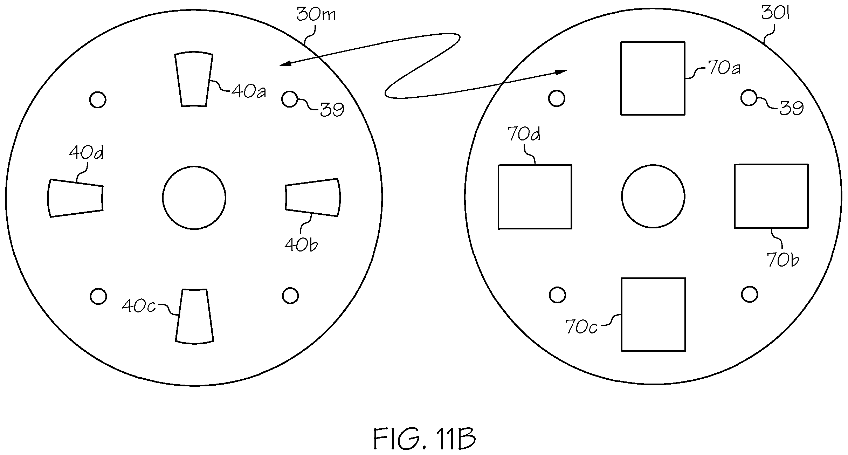

FIG. 11B is a top view of an embodiment of a sample subunit and a corresponding source subunit, in accordance with aspects of the present inventive concepts.

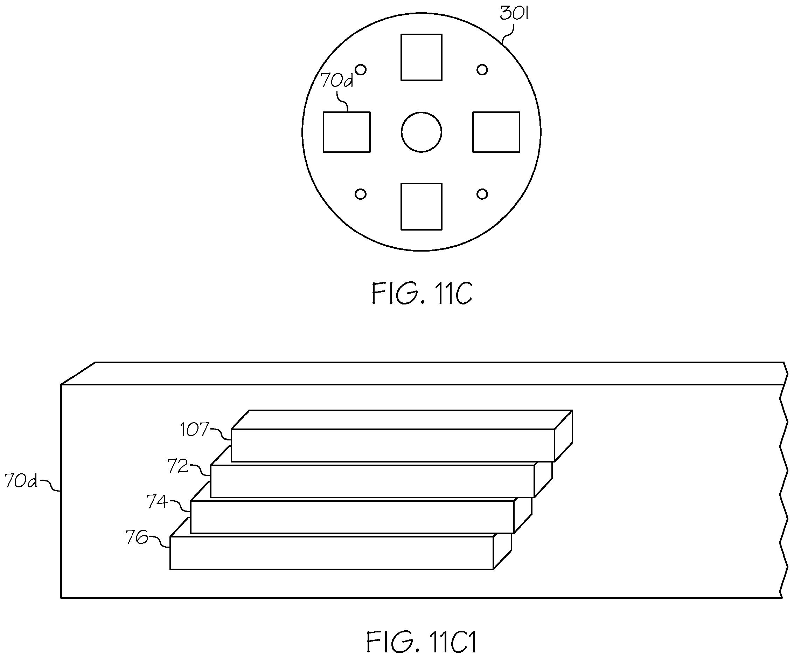

FIG. 11C is a top view of an embodiment of a source subunit, in accordance with aspects of the present inventive concepts.
FIG 11C1 is a close-up perspective view of an embodiment of one or more illumination elements 70 at a position at a source subunit, in accordance with aspects of the present inventive concepts.

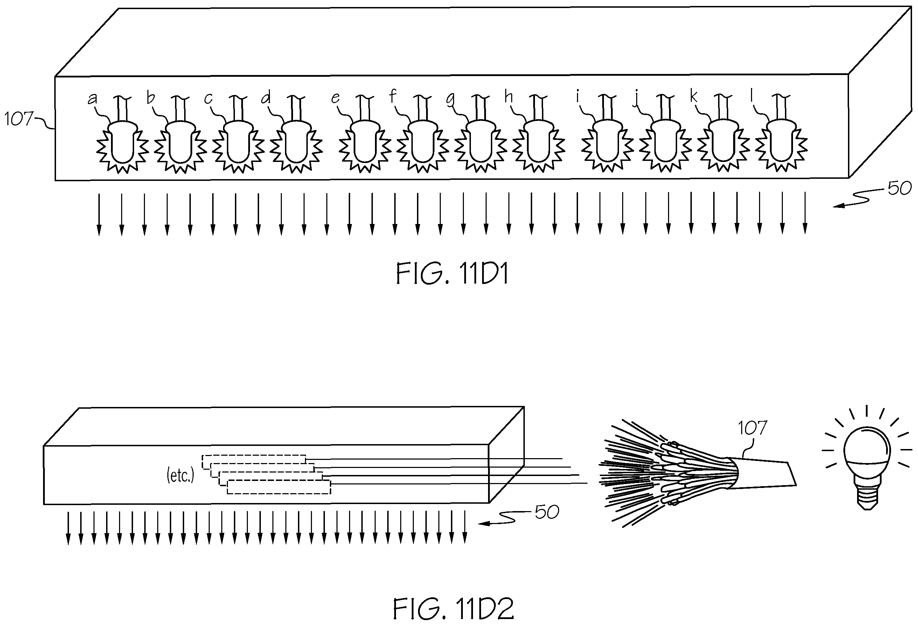

FIG. 11D1 is a perspective view of an embodiment of sources of electromagnetic radiation, in accordance with aspects of the present inventive concepts.
FIG. 11D2 is a perspective view of an embodiment of sources of electromagnetic radiation, in accordance with aspects of the present inventive concepts.

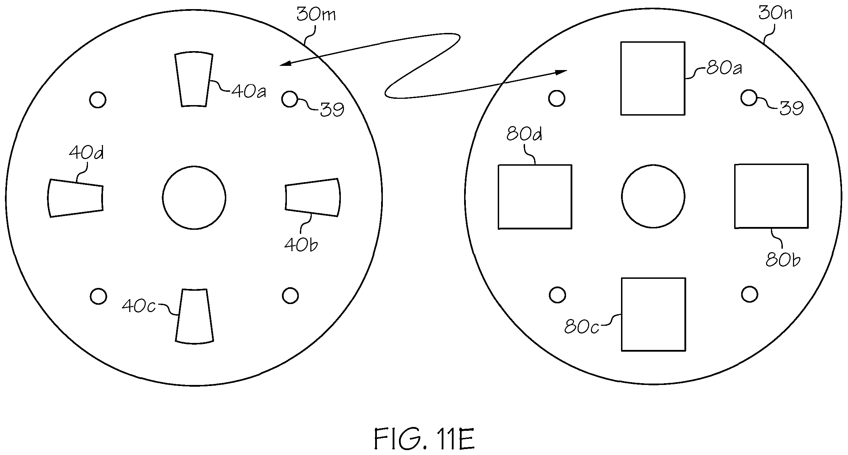

FIG. 11E is a top view of an embodiment of a sample subunit and a corresponding detection subunit, in accordance with aspects of the present inventive concepts.

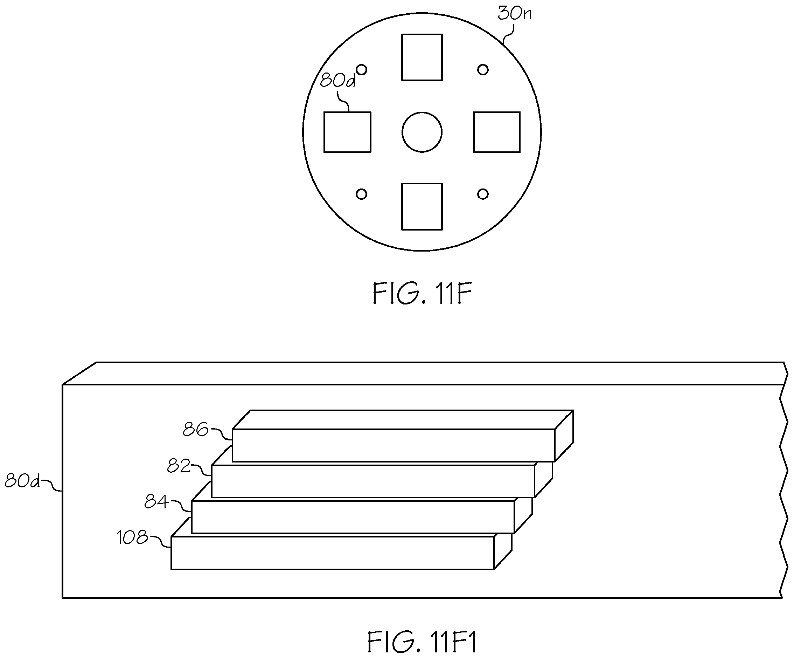

FIG. 11F is a top view of an embodiment of a detection subunit, in accordance with aspects of the present inventive concepts.
FIG 11F 1 is a close-up perspective view of an embodiment of one or more detection elements, in accordance with aspects of the present inventive concepts.

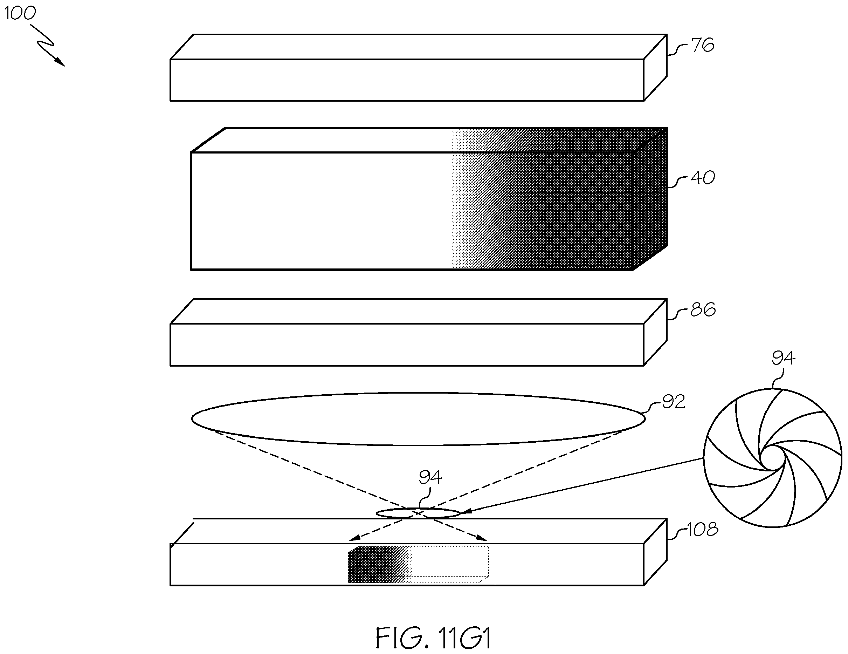

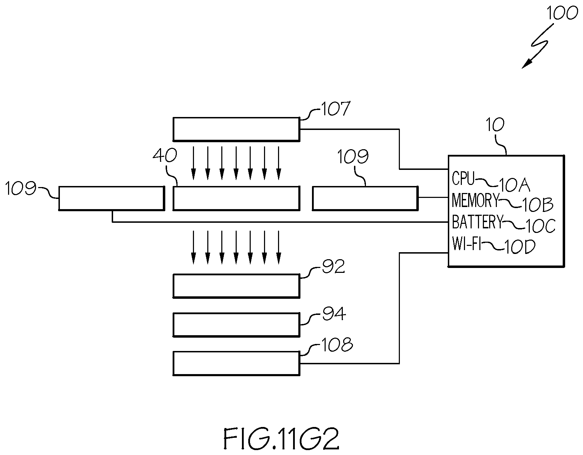

FIG. 11G1 is a perspective view of an embodiment of a rotor system comprising a Schlieren image detection system, in accordance with aspects of the present inventive concepts.
FIG. 11G2 is a conceptual diagram of an embodiment of a rotor system configured to perform Schlieren imaging, in accordance with aspects of the present inventive concepts.

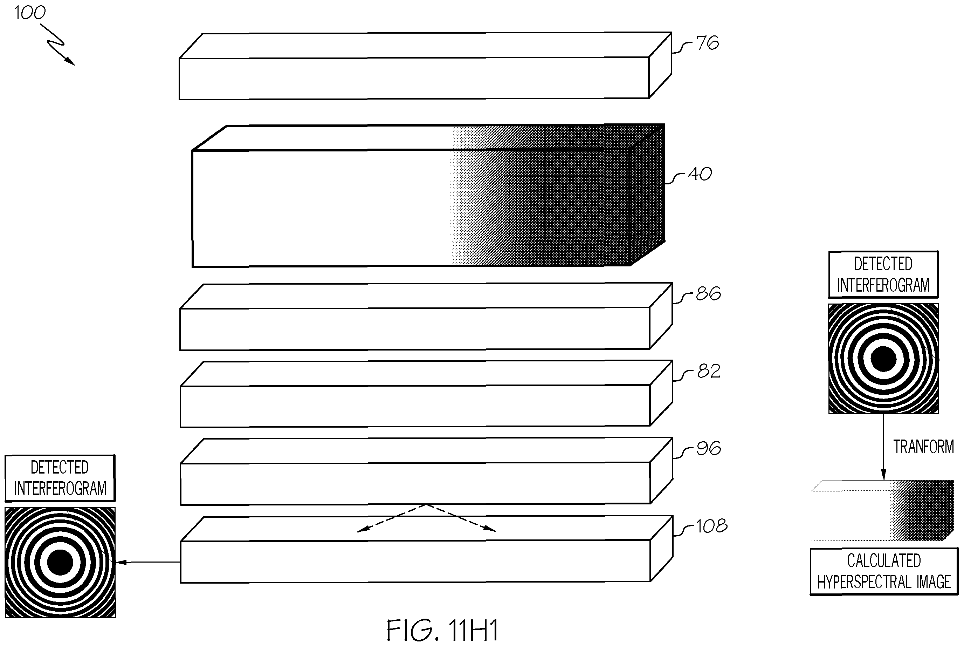

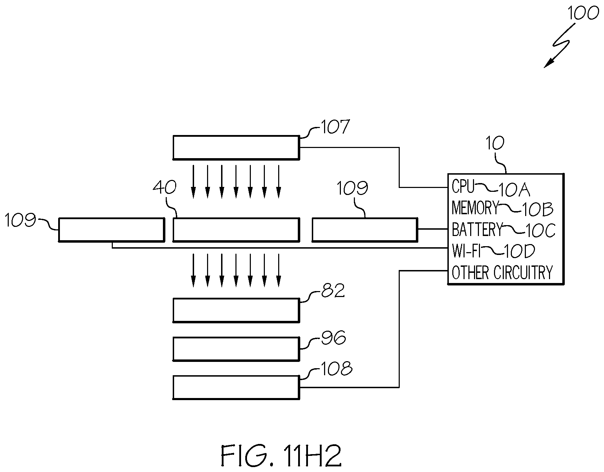

FIG. 11H1 is a perspective view of an embodiment of a rotor system comprising a hyperspectral image detection system, in accordance with aspects of the present inventive concepts.
FIG. 11H2 is a conceptual diagram of an embodiment of a rotor system configured to perform hyperspectral imaging, in accordance with aspects of the present inventive concepts.

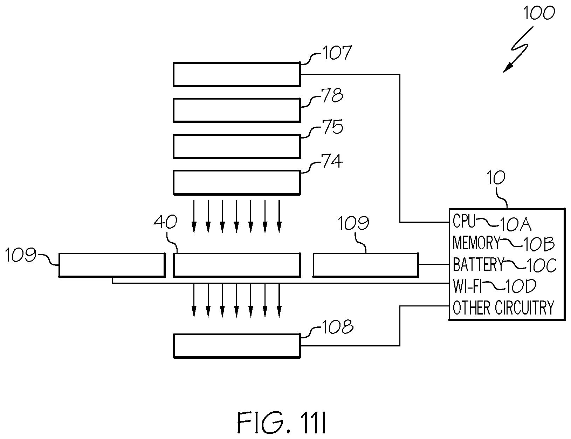

FIG. 11I is a conceptual diagram of an embodiment of a rotor system with a filtered illumination source, in accordance with aspects of the present inventive concepts.

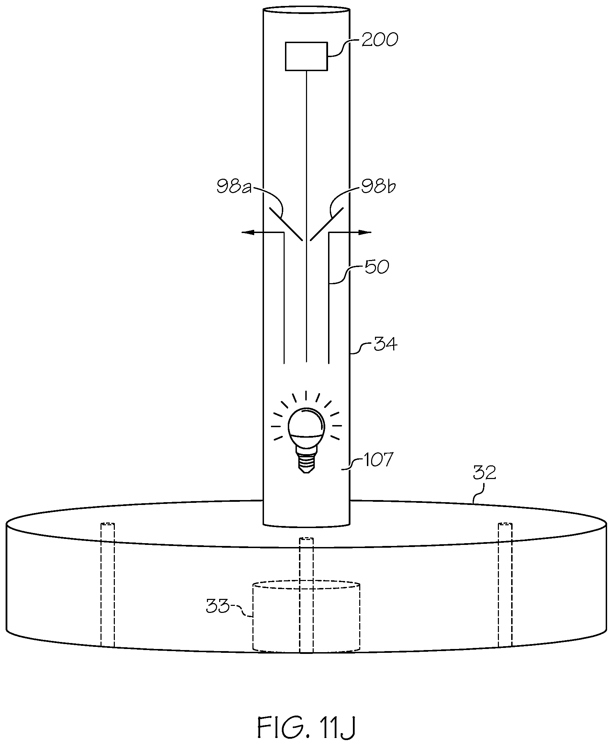

FIG. 11J is a perspective view of an embodiment of a central projection comprising a source of electromagnetic radiation, in accordance with aspects of the present inventive concepts.

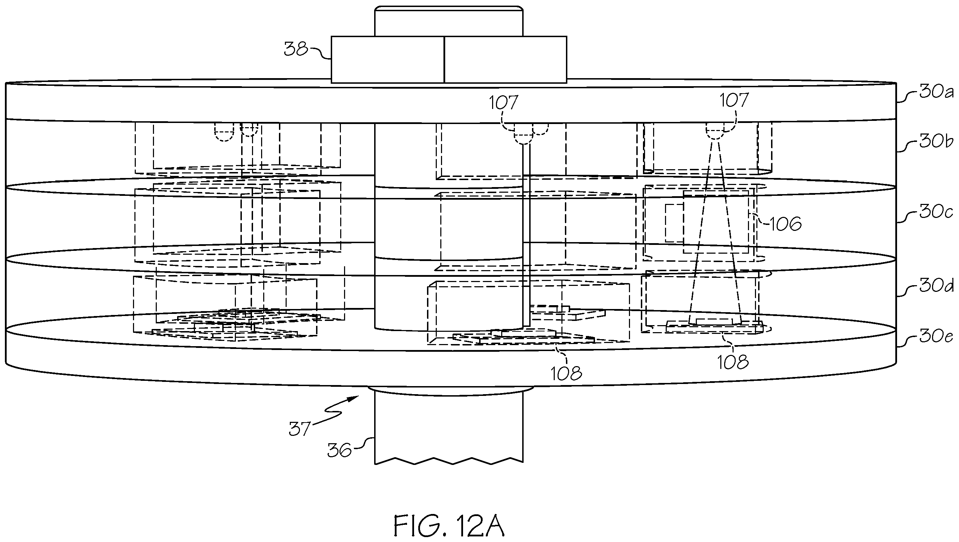

FIG. 12A is side view of an embodiment of a rotor system, in accordance with aspects of the present inventive concepts.

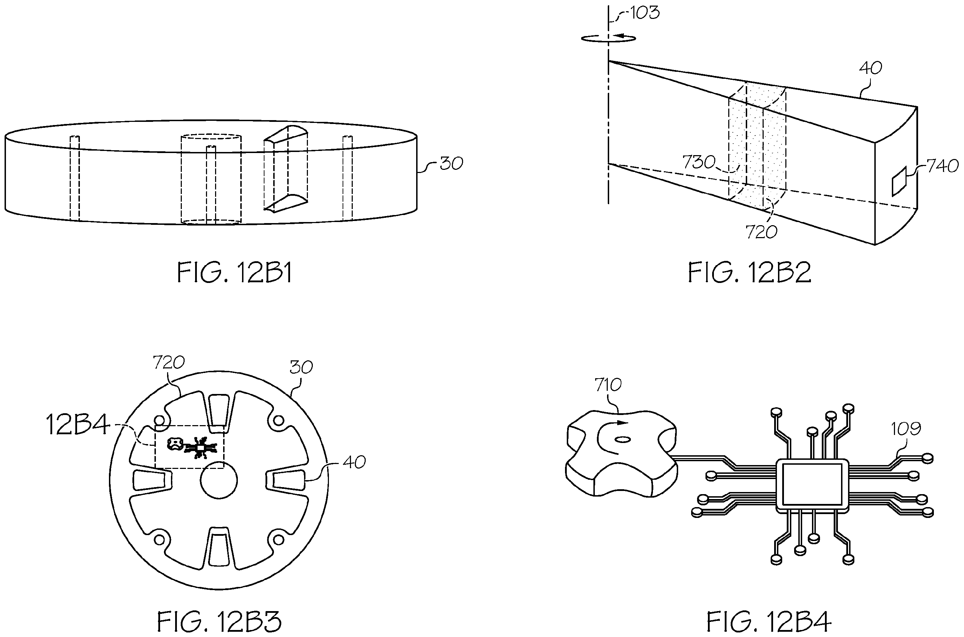

FIG. 12B1 is a perspective view of an embodiment of a subunit comprising at least one sample region and a temperature control system, in accordance with aspects of the present inventive concepts.
FIG. 12B2 is a close-up perspective view of an embodiment of a sample region from FIG. 12B1, in accordance with aspects of the present inventive concepts.
FIG. 12B3 is a top view of an embodiment of a sample subunit comprising a thermal channel, in accordance with aspects of the present inventive concepts.
FIG. 12B4 is a close-up perspective view of an embodiment of a temperature control element and a stirrer, in accordance with aspects of the present inventive concepts.

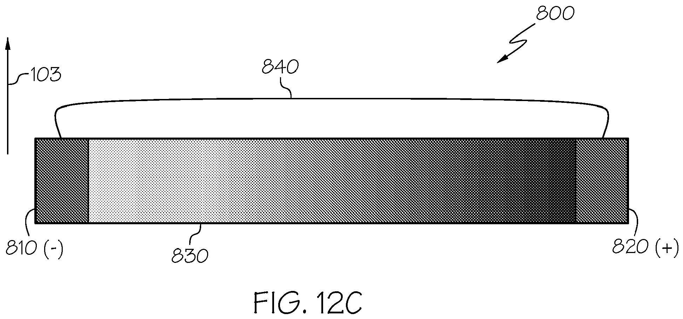

FIG. 12C is a side view of an embodiment of a centrifugally-driven voltaic cell constructed and arranged to provide electrical power to one or more subunits, in accordance with aspects of the present inventive concepts.

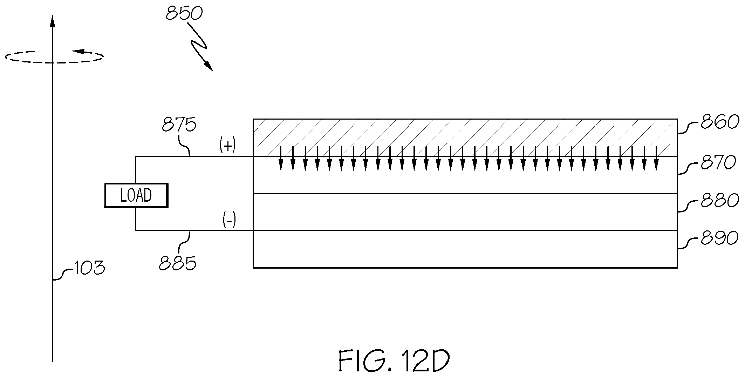

FIG. 12D is a side view of an embodiment of a voltaic cell constructed and arranged to be driven by beta particle emission from radio-isotope decay, to provide electrical power to one or more subunits, in accordance with embodiments of the present inventive concepts.

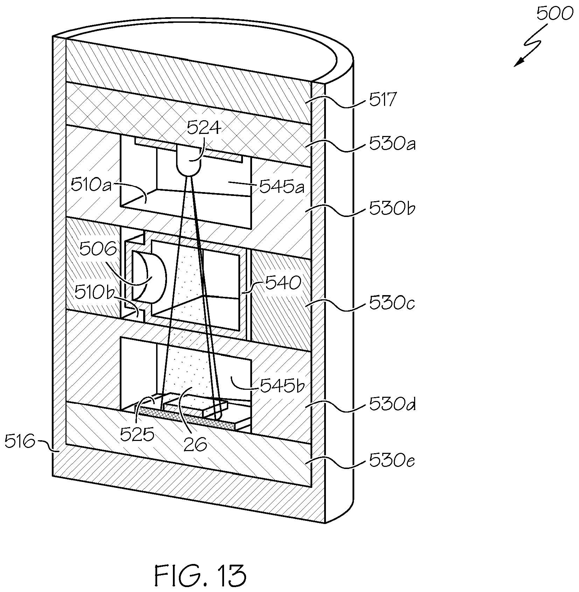

FIG. 13 is a perspective sectional view of an embodiment of an interrogation cell, in accordance with aspects of the present inventive concepts.

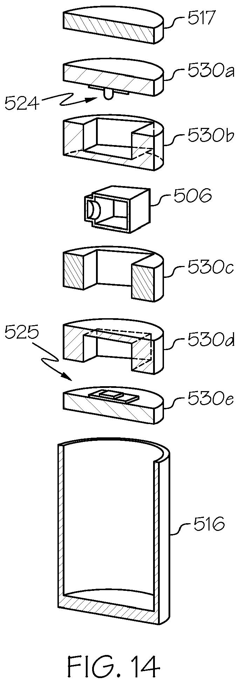

FIG. 14 is an exploded sectional view of an embodiment of an integrated interrogation cell device, in accordance with aspects of the present inventive concepts.

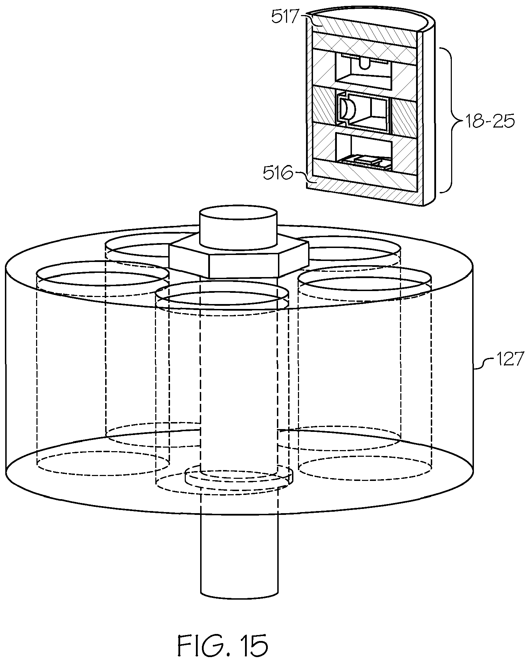

FIG. 15 is a perspective sectional view of an embodiment of an assembled cell device, in accordance with embodiments of the present inventive concepts.

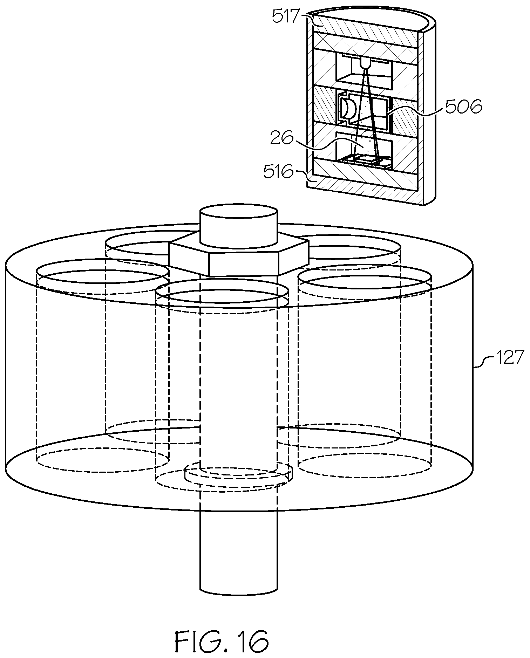

FIG. 16 is perspective sectional view of an embodiment of an assembled cell device, in accordance with aspects of the present inventive concepts.

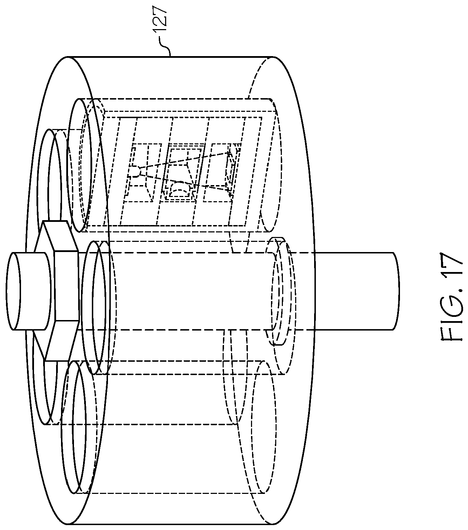

FIG. 17 is a perspective view of an embodiment of an assembled cell device fitted inside a rotor, in accordance with aspects of the present inventive concepts.

### DETAILED DESCRIPTION

Reference will now be made in detail to the present embodiments of the technology, examples of which are illustrated in the accompanying drawings. Similar reference numbers may be used to refer to similar components. However, the description is not intended to limit the present disclosure to particular embodiments, as the scope of protection is defined by the appended claims.

It will be further understood that, although the terms first, second, third etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on", "attached", "connected" or "coupled" to another element, it can be directly on or above, or connected or coupled to, the other element, or one or more intervening elements can be present. In contrast, when an element is referred to as being "directly on", "directly attached", "directly connected" or "directly coupled" to another element, there are no intervening elements present. Other words used to describe the relationship between elements should be interpreted in a like fashion (e.g. "between" versus "directly between," "adjacent" versus "directly adjacent," etc.).

As used herein, the term "proximate" shall include locations relatively close to, on, in and/or within a referenced component, anatomical location, or other location.

Spatially relative terms, such as "beneath," "below," "lower," "above," "upper" and the like may be used to describe an element and/or feature's relationship to another element(s) and/or feature(s) as, for example, illustrated in the figures. It will be further understood that the spatially relative terms are intended to encompass different orientations of the device in use and/or operation in addition to the orientation depicted in the figures. For example, if the device in a figure is turned over, elements described as "below" and/or "beneath" other elements or features would then be oriented "above" the other elements or features. The device can be otherwise oriented (e.g. rotated 90 degrees or at other orientations) and the spatially relative descriptors used herein interpreted accordingly.

The terms "reduce", "reducing", "reduction" and the like, where used herein, are to include a reduction in a quantity, including a reduction to zero. Reducing the likelihood of an occurrence shall include prevention of the occurrence.

The term "and/or" where used herein is to be taken as specific disclosure of each of the two specified features or components with or without the other. For example, "A and/or B" is to be taken as specific disclosure of each of (i) A, (ii) B and (iii) A and B, just as if each is set out individually herein.

It is to be understood that at least some of the figures and descriptions of the disclosure have been simplified to focus on elements that are relevant for a clear understanding of the disclosure, while eliminating, for purposes of clarity, other elements that those of ordinary skill in the art will appreciate may also comprise a portion of the disclosure. However, because such elements are well known in the art, and because they do not necessarily facilitate a better understanding of the disclosure, a description of such elements is not provided herein.

An integrated rotor device for autonomous analytical centrifugation offers advantages over former approaches in that the rotating device itself is equipped with all the means to complete an AUC assay, with the exception of the centrifugal force, in contrast to the previous art where the majority of the measurement functions are carried out on modules located within the centrifuge, rather than by assembling the critical components within the confines of a rotor. Therefore, by locating most of the assay functionality into self-contained modules or cells within a spinning rotor apparatus, the embodied device is versatile and efficient, and can be used with a range of centrifuges including smaller, cost effective benchtop (table-top) centrifuges where lower centrifugal speeds are sufficient, such as in the analysis of high molecular weight biological species including viral particles and gene delivery vectors. Furthermore, by having the light source, sample, and detector all move together (in register) during sedimentation, in the rotating frame of reference, the speed of data acquisition, and the quality of data, are improved, resulting in higher resolution and precision in the numerical results.

In this manner, a system is provided that is portable, cost-effective, and versatile in its measurement capabilities, and that allows for the collection of high quality sedimentation profile data.

In an embodiment of the device, cells can be loaded into the rotor without the need for any special cell alignment, as such cell alignment can be highly subjective, based on interpretation by the user operating the equipment.

In another embodiment, numerical output of the sedimentation data can be provided in a retrievable digital format, following a minimum of input parameters required of the technician operating the device.

In another embodiment, numerical output of the sedimentation data can be provided in a retrievable digital format, in real-time during the sedimentation, by means such as Wi-Fi, Bluetooth, or other wireless mode for transmission of the data to an external unit or to Cloud Storage.

In another embodiment, robust temperature control of the fluid chamber can be provided by thermostatic devices (such as thermoelectric units) placed internally in the rotor device and nearby the fluid-chamber region of the device.

In another embodiment, high speed data acquisition, and improved data quality, can be provided by collecting light absorbance, interference, or fluorescence profiles across the radial dimension of each fluid chamber simultaneously along said radial dimension, via the use of a diode array, rather than sequentially, via the use of a detector on a radially moving armature system.

In another embodiment, an improvement in the quality of AUC data can be provided by collecting light absorbance, interference, or fluorescence profiles across the radial dimension of the fluid chamber while the source and detector remain fixed in the same frame of reference as the fluid chamber (the rotating frame of reference), during centrifugation and data collection.

A measurement device is in the form of an assembly of integrated modules and/or functional cells, within a rotor, and contains-internal to the rotor-all provisions for the acquisition of sedimentation profile data in the manner of a controlled AUC assay.

In some embodiments, a fluid chamber region confines the sample and/or reference fluids in a sector-shaped geometry. The fluid chamber is a walled unit, or multiple such units, that are open on the top and bottom faces and are bounded on the top and bottom faces by transparent windows that may be affixed to each open face of the chamber, sealing the unit in all directions against leakage, and allowing the passage of electromagnetic radiation, such as visible or UV light, and other types of sample monitoring.

An assembly of integrated modules or functional cells within a rotor body, is configured with the rotor body having an opening or cavity that is coincident with the axis of rotation so that the rotor may be placed atop the drive shaft (spindle) of a centrifuge, in a manner similar to conventional analytical rotors (i.e. non-functional or non-data acquiring rotors which are simply structural units composed of e.g. solid titanium). The cavity is configured to accept the drive shaft and with proper adaptation may be acceptable to a plurality of shaft dimensions.

A centrifuge, which is external to, and separate from the rotor device, and in which the rotor device is placed, provides centrifugal force which causes sedimentation in the sample and/or reference chambers. In some embodiments, a source of electromagnetic radiation (light source) and a detector are affixed along a linear axis that is parallel to the axis of rotation and coincident with a straight line that traverses the transparent windows that bound the top and bottom faces of the fluid chamber.

In some embodiments, a separate light source and detector are placed along the linear axis through each separate fluid chamber that is housed within a cell structure and/or within the entire rotor device. In some embodiments, the light source and detector are rigidly affixed within the rotor device, so as to maintain the co-linear positioning of the light source, fluid chamber, and detector at all times before, during, and after centrifugation; in this manner each fluid chamber may be illuminated, and sedimentation subsequently detected, simultaneously across the radial dimension of each sample/reference chamber, and likewise each separate fluid chamber may also be illuminated and detected simultaneously with (and/or independently of) any other chamber.

In some embodiments, the light source, detector, and other modules stacked together in a rigid sleeve or housing, which may be considered a functional cell, which can be removed from the rotor, and then rigidly affixed to back to the rotor at appropriate times prior to the start of an assay.

The source of radiation associated with each chamber may be a single diode or an array of light emitting diodes that uniformly illuminate the radial dimension of each chamber, and the detector can likewise be a corresponding array of diodes, charge coupled devices, or the like, which develop a voltage or current as a function of illumination. The respective voltages or amperages can be shifted to a threshold detector means for encoding light intensity levels at the pixels and comparing the intensity levels for resolving the sedimentation profiles in each chamber at prescribed times throughout the sedimentation. The radiation and detection functions are managed and controlled by a microprocessor and appropriate circuitry and chipsets, to likewise control and maintain the desired chamber temperature, process the samples accurately in a programmed manner, and calculate and display the results numerically.

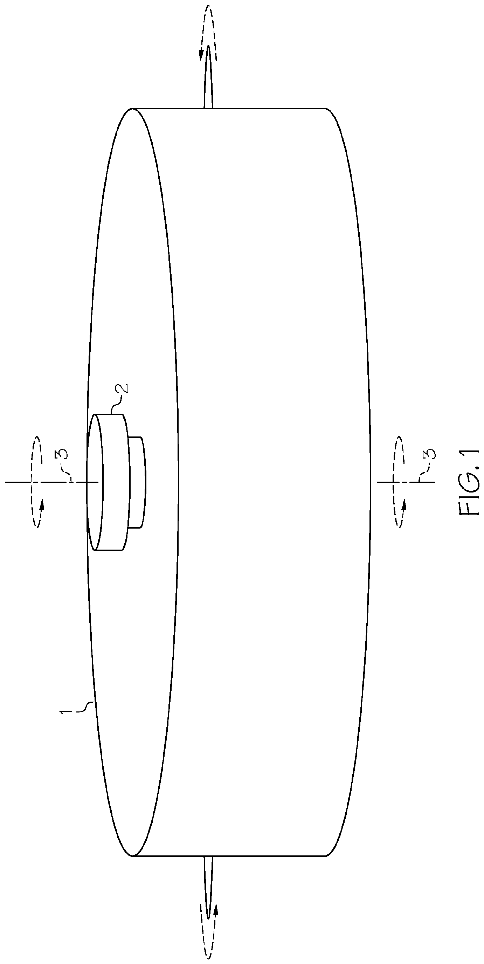

FIG. 1 is a perspective view of an integrated rotor device having rotor body 1, or chassis, in accordance with embodiments of the present inventive concepts. In some embodiments, the integrated rotor body 1 is constructed from a rigid material, and having a top handle feature 2. The form of the body 1 and handle 2 is symmetric with respect to an axis of rotation 3 which passes through the center of the device. Referring to FIG. 1, in some embodiments, the main body of the rotor device 1 has a cylindrical shape. In other embodiments, the body 1 may take other forms that are not purely cylindrical, but are nevertheless symmetric about the axis of rotation.

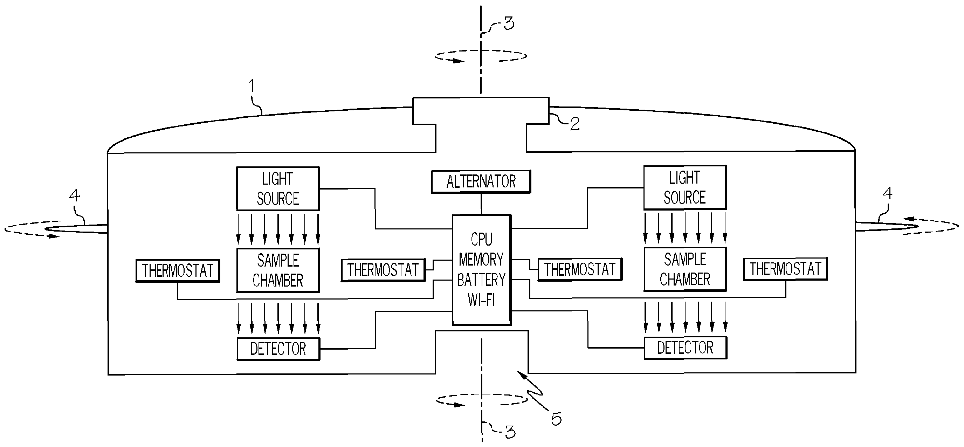

FIG. 2 is a perspective view having a cutaway section through the rotor body 1 in accordance with embodiments of the present inventive concepts. The cutaway section is provided in this drawing to illustrate the possible positioning of critical components which are internal to the rotor body 1, and because of the rigid nature of the chassis, these internal components are designed to all move in register with the plane of rotation 4, which correlates with the rotating axis 3. The rotational force which spins the chassis 1 about the axis 3 (and therefore likewise the plane 4) is, in some embodiments, provided by an external centrifuge which the embodied device is designed to interface with via the spindle mating cavity **5,** which mates with the spindle or drive shaft of the external centrifuge. In some embodiments, the cavity **5** is configured to be adaptable so as to accept a plurality of shaft dimensions that may be common to many commercial centrifuges.

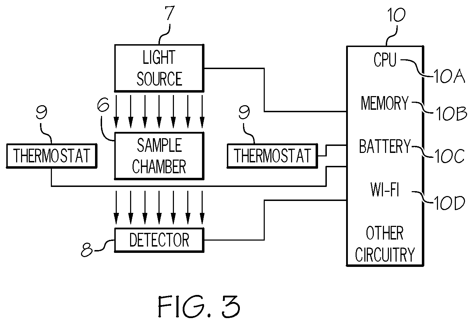

FIG. 3 is a diagram which illustrates, and individually labels, several components which may be contained within the rigid body **1** of the rotor and depicts a possible relative orientation and connectivity of the components to form a system capable of measuring sedimentation data in an autonomous device. The sector-shaped sample chamber **6** is represented as a central component surrounded by a light-source **7** and detector **8,** which in this embodiment are depicted as above and below the sample chamber respectively.

The depiction of FIG. 3 shows only one such sample chamber **6,** although in some embodiments, multiple chambers **6** may be housed within the rotor body **1,** in a symmetrical fashion, with attendant modules (including, but not limited to, light source **7** and detector **8**) likewise symmetrically orientated and properly interconnected.

The depiction of FIG. 3 refers to the above/below orientations, which are with respect to the plane of rotation **4.** The plane of rotation is defined as normal to the axis of rotation **3,** and the plane is defined as passing through the center of the sample chamber or chambers, although other arrangements and/or pluralities of the light source(s) **7** and detector(s) **8** are possible depending on the nature of the radiation and the detection strategy.

Also surrounding each sample chamber **6** are thermostat units **9** which may be thermoelectric circuit components, and may be located in close proximity to the sample chamber **6** for the purpose of maintaining a constant temperature in the region where the sample fluid resides, and in particular in the fluid itself, to buffet against any temperature gradients which may arise from irradiation of the fluid or nearby circuitry, or other sources. Although not detailed in FIG. 3, the thermostat units **9** may have in some embodiments accompanying heat sinks to augment the thermostatic function of the units labelled as **9.**

Finally in FIG. 3 is shown possible electrical interconnectivity between the units **7, 8,** and **9**, and a circuit board, or boards, **10**, which contain critical components such as the CPU (**10A**), digital memory such as RAM/ROM (**10B**) or removable micro SD, on-board rechargeable battery (**10C**), wireless communication circuitry (**10D**), and other components necessary for the ability to control and manage the functioning of the attendant modules in a programmable manner and for the proper collection, storage, and retrieval of sedimentation data. Notably, FIG. 3 depicts one possible interconnectivity such as via wiring, but other inter-connectivities are possible to accept, for example, a plurality of measurement components around multiple sample chambers **6,** which in some embodiments may all be connected to a single main CPU.

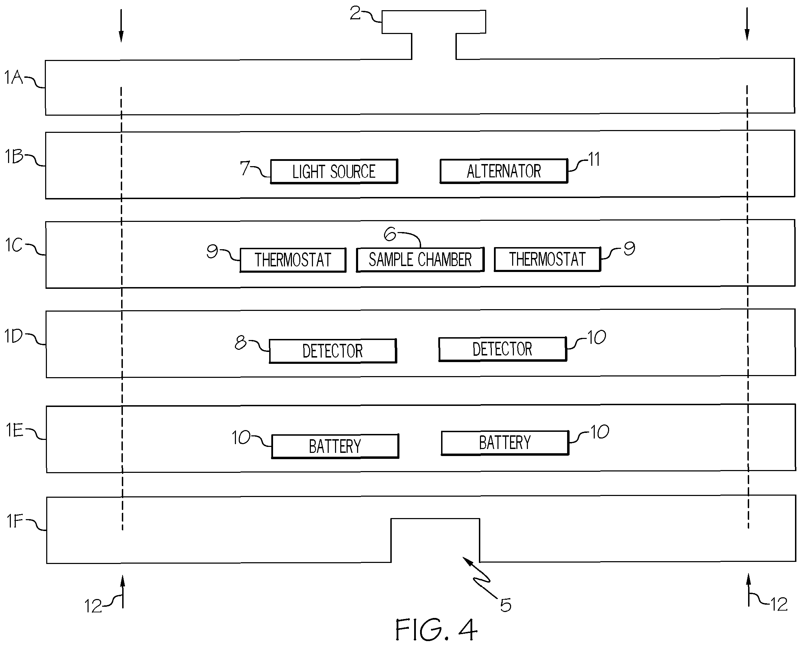

FIG. 4 is a side elevation schematic of an integrated rotor device capable of modular assembly/disassembly, in accordance with embodiments of the present inventive concepts. The arrangement of functional units **6, 7, 8, 9, 10,** which were introduced in FIG. 3 and which are necessary for the autonomous collection of sedimentation data, are, in the embodiment of FIG. 4, shown as located on separated parts of a rotor body **(1A, 1B, 1C,** etc.). The separation of the rotor body **1,** into separable units, which may be assembled into one rigid body, provides a means by which to locate functional units such as **6, 7, 8, 9, 10** in a way that the units can be cleaned, maintained, modified, interchanged, or otherwise manipulated prior to final assembly in preparation for an assay. Introduced in FIG. 4 is an embodiment wherein a functional unit such as an alternator **11,** that is a type of electrical generator, is present and which may be capable of converting a fraction of the rotational energy of the spinning rotor, during an assay, into electrical current which may provide supporting power for the autonomous device during an assay, and/or may be capable of recharging the battery based on the charge depletion state of the battery at any given time. Beginning from the disassembled state, with all rotor parts **1A, 1B, 1C,** etc., separated, once all functional units are properly adjusted and ready for analysis, the rotor parts **1A, 1B, 1C,** etc., are then rigidly affixed together, for example as indicated in this embodiment by a fastening mechanism, represented by **12,** such as, but not limited to, tensioning rods, screws, or bolts.

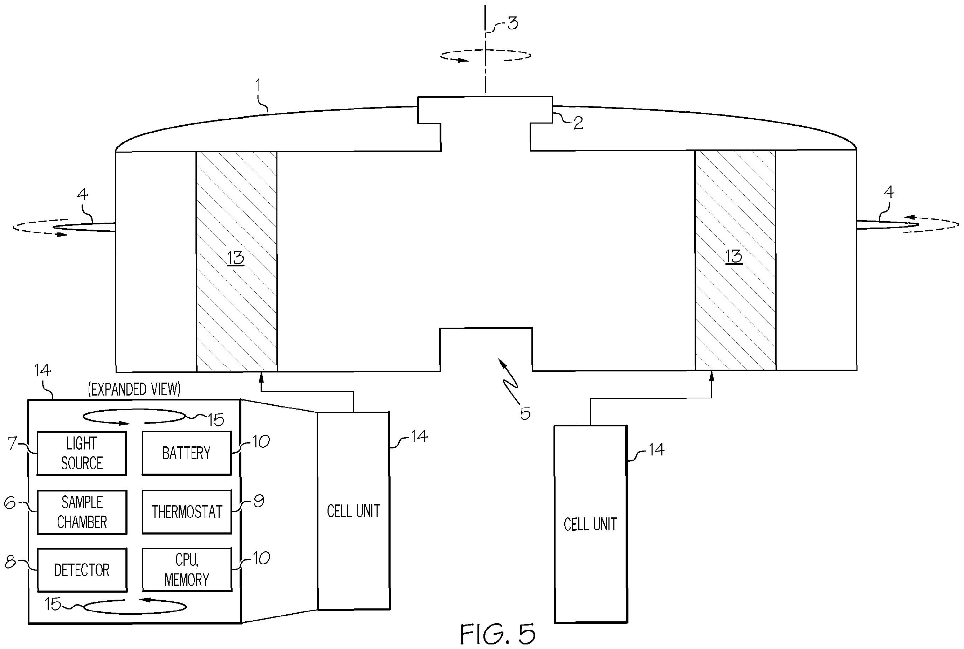

FIG. 5 is a perspective view having a cutaway section, in accordance with embodiments of the present inventive concepts. The embodiment of FIG. 5 emphasizes a device whereby the critical functional components such as **6, 7, 8, 9, 10,** are located in a smaller geometry, specifically, a functional cell-like unit. The embodiment of FIG. 5 shows a rotor body **1** configuration having cylindrical cavities that are parallel to the axis of rotation **3,** and that are arranged symmetrically about the axis. Although only two such cavities **13** are shown, a multitude of cavities may in fact be realized within any single rotor body **1.** Given that sufficient miniaturization of the functional components is possible, the components **6, 7, 8, 9, 10** may all be co-located onto a small structural unit which may be designated as a cell **14.** The components within the cell may be rigidly affixed within the cell body via the use of a tensioning mechanism **15,** such a screw rings. Once assembled each cell unit **14** can then be affixed inside a corresponding cavity **13,** and the rotor device is prepared for data collection.

Furthermore, the cell units **14** embodied in FIG.5 may be sufficiently miniaturized such that the cell unit dimensions are comparable to that of AUC cells already in use for conventional AUC experiments (i.e. conventional AUC cells that have no internal functionality but rather simply just contain the sample in a fixed geometry). In such cases the cell units **14** may be compatible with analytical rotors already in use and can be affixed inside such commercially available rotors for the generation of autonomous sedimentation data.

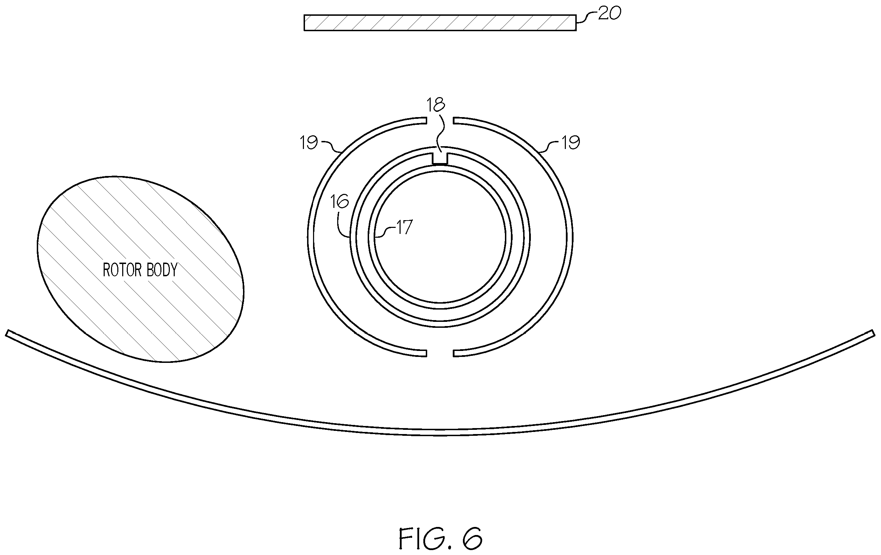

FIG. 6 is a top view of an embodiment in accordance with the present inventive concepts. In some embodiments, the cavity wall **16** comprises a collar **17** which is a ridge or extension of the wall. The collar **17** may form a ring around an axis that extends through the center of the optical cavity. In some embodiments, the collar **17** is at a fixed height in the optical cavity. The purpose of the collar **17** is to provide a stop against which components will press against, and thus components can be rigidly secured inside the cavity.

A key-way **18** extends along the cavity wall in a direction parallel to the axis of rotation. The key-way **18** is constructed and arranged to mate with components that are stacked inside the optical cavity, to provide precise and robust alignment of the components with respect to the axis of rotation. Each key-way **18** inside each cavity may be removable or may be a rigid, continuous part of the cavity wall, as may be desired for precise and robust alignment and positioning of the stacked components. From the top view, the key-way **18** appears as a ridge protruding into the optical cavity, and extends the length of the cavity from top to bottom.

One or more thermostat devices **19,** such as thermoelectric modules, may be positioned around the optical cavity. The one or more thermo-electric devices **19** may be rectangular in shape having some fixed thickness. In some embodiments, the thermo-electric devices **19** may be curved, so as to surround the optical cavity to better maintain the temperature in the vicinity of one or more sample/reference chambers. In the embodiment shown in FIG. 6, the thermostat (thermoelectric) modules **19,** are card-like in form, and run parallel to the length of the cavity from top to bottom. In the embodiment shown, the modules **19** are intended to fit into slots in the rotor body 1, and can be inserted, removed and replaced from the slots.

In some embodiments, the integrated rotor device contains a card-slot **20.** The card-slot **20** may be constructed and arranged to couple with an electric circuit board. In some embodiments, the circuit board is coupled to the one or more thermo-electric devices **19** by any suitable means, such as ribbon-cable or cable-cord connectors. Measurement components (including, but not limited, to the light sources and detectors) may also be coupled to the circuit board. The circuit board may comprise a central microprocessor unit (CPU). The circuit board may further comprise other chip sets and circuit components, including, but not limited to, digital memory modules, rechargeable batteries for supplying power, and overall management of the measurement and temperature control in a programmable manner. Although FIG. 6 shows one card-slot **20** constructed and arranged to couple with a circuit board, other embodiments may comprise a different number of card-slots and circuit boards. The number of card-slots and circuit boards depends on the computing, data management, and power needs.

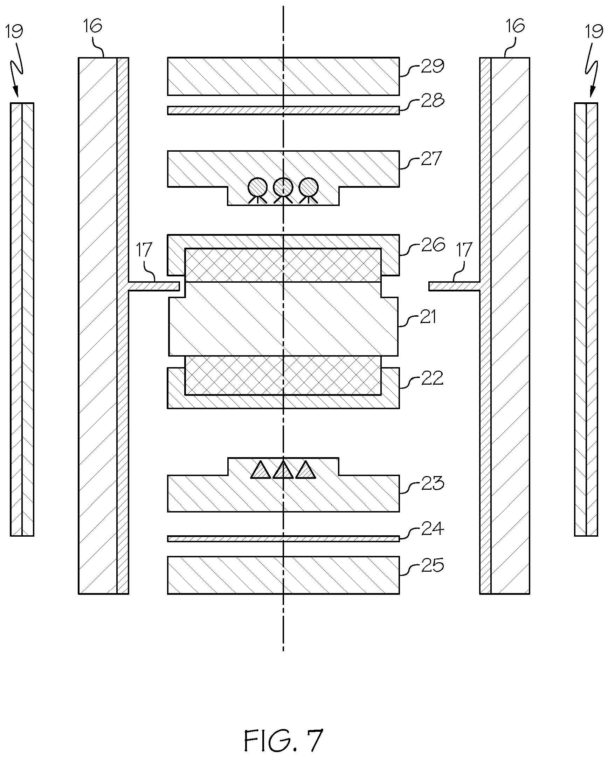

FIG. 7 is a section view of an embodiment of an optical cavity in accordance with the present inventive concepts. The cavity walls **16** are shown, as well as the collar **17,** which was previously described. Thermostat devices, such as thermoelectric modules, **19** are also shown on both sides of the optical cavity.

The integrated rotor device may comprise a centerpiece **21.** In some embodiments, the centerpiece **21** is a walled unit that encloses sector-shaped compartments of the sample and/or reference solutions (fluids).

Specifically, a sector-shaped geometry is defined as the volume element of a cylindrical segment having the cross-sectional area of a circular sector (an area enclosed by two radii of a large circle, an arc of the larger circle, and an arc of a smaller concentric circle of lesser radius).

In some embodiments, the centerpiece **21** is inserted from the bottom of the rotor, up through the cavity walls, and the centerpiece is pushed upwards until it meets with the collar **17.** In some embodiments, the centerpiece comprises a first ridge on an upper surface that couples with the collar and allows the upper surface of the centerpiece **21** to protrude slightly above the collar **17.** The centerpiece **21** may include a second ridge that allows it to couple with the key-way **18,** for precise alignment of the centerpiece **21** in the optical cavity.

In some embodiments, a bottom window unit **22** is inserted from the bottom of the optical cavity. In some embodiments, the bottom window unit **22** is a rigid metal housing into which a transparent (quartz or the like) cylindrical window is held. The bottom window unit **22** may also have a key-way mating ridge as part of its structure, for precise and optimal alignment of the window. In the embodiment shown in FIG. 7, the bottom window unit **22** is pushed upwards until the window surface is in full, flush contact with the lower surface of the centerpiece **21.**

In some embodiments, a detector unit **23** is inserted from the bottom of the optical cavity and pushed upwards in a similar manner. The detector unit **23** can also contain a key-way mating ridge as part of its structure, for the same purposes as described above for the centerpiece, etc. The detector **23** may be of a certain geometry such that part of the detector device may protrude upwards and allow it to mate with an opening in the bottom window holder **22** structure, allowing for close contact between parts of the detector and the (e.g. quartz) window surface.

In some embodiments, the detector unit **23** is constructed and arranged to resolve the differential absorbance (or other signal) that is spread out over the radial dimension in the sample chamber, as sedimentation occurs during centrifugation. In some embodiments, the detection is accomplished by a linear arrangement of an array of detector units, such as an array of diodes that can detect the intensity of light, or other detection means such as fluorescence, refraction, etc. Because the detector **23** is affixed in register with the sample chamber, the detector **23** is designed to experience the same centrifugal velocity as the sample chamber. In some embodiments, the detector **23** can be programmed to acquire data at any desirable time during centrifugation, simultaneously across the radial dimension and independently of any other sample chamber being centrifuged in the same rotor. In some embodiments, the detector **23** is designed to transmit it signals, such as voltages or current values, by means of a wire connect to the card-slot peripheral **20** (refer to FIG. 6).

In some embodiments, a bottom gasket **24** is pushed up from the bottom of the optical cavity to interface with the bottom face of the detector unit **23.** In some embodiments, a bottom screw ring **25** is also affixed from the bottom, which mates with threads in the wall of the optical cavity. The purpose of the bottom screw ring **25** is to provide a significant tension force that can be adjusted and made to press together all the subunits of the bottom half of the cavity, which press against the collar **17.** Once sufficient tension force is applied via the bottom screw ring **25,** the bottom half of the subunits provide a leak-tight junction between the inner walls of the centerpiece **21** (the sample/reference chambers) and the bottom face of the centerpiece which meets flush with the window surface.

In some embodiments, the sample and/or reference fluids may be pipetted into the open face of the fluid-tight chambers from above, i.e. the sector shaped chambers of the centerpiece **21.** Note that by first sealing the centerpiece and the bottom window by providing tension against the collar, the sector-shaped chamber is fluid-tight and can be filled with a certain volume of fluid via top of the chamber which remains open until the top window is applied.

In some embodiments, after inserting the fluids, a top window assembly **26** is placed down over the centerpiece **21.** In some embodiments, the top window assembly **26** is aligned with the key-way **18** (FIG. 6). Because the top ridge of the centerpiece **21** extends just above the elevation of the collar **17,** a flush contact between centerpiece **21** and a top window **26** is accomplished. Once tension from the top is applied, at such point both the top and bottom faces of the centerpiece are sealed, and the fluid is at that point confined in a chamber which is leak tight in all dimensions.

In some embodiments, a light source **27** is inserted down into the cavity. In some embodiments, the light source **27** comprises a key-way ridge for alignment. In some embodiments, the light source **27** is constructed and arranged to provide uniform sample illumination. The light source **27** may be a monochromatic or narrow band wavelength or may be a broadband source that is made monochromatic by sufficient means for transmission through the light source module.

Similar to the bottom half of the cavity assembly, the top portion may be sealed by means of a top gasket **28** and a top screw ring **29.** Once the top screw ring **29** is fully torqued down, the entire stack is a fluid-tight and air-tight stack enclosed in the rotor body. Because there are electrical connections between the light source-CPU and detector-CPU, the illumination and subsequent detection across the sample chamber can be operated in a controlled and programmed manner. The input of experimental parameters is intended to be entered by the operator prior to the placement of the device in the centrifuge, by means of a separate electrical connection between the device and a workstation, such as by USB connection.

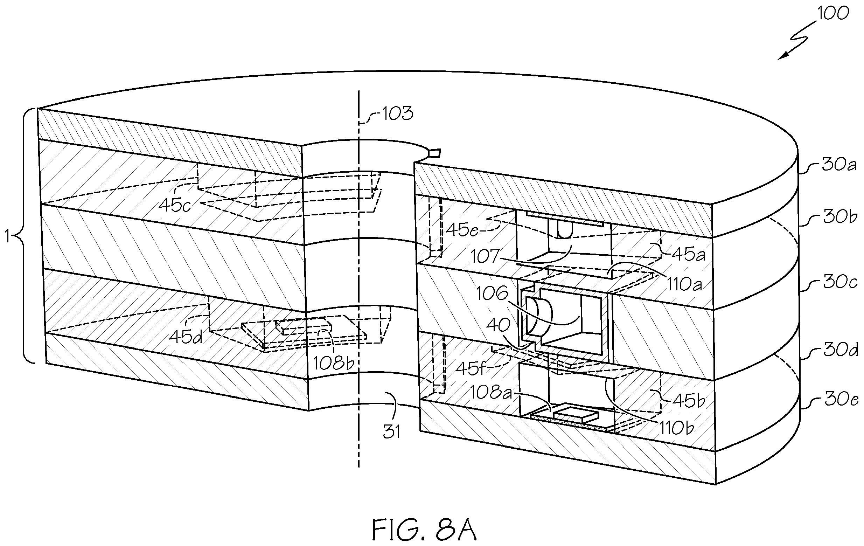

FIG. 8A is a perspective sectional view of an embodiment of a rotor system 100 (also referred to as a Smart Rotor) in accordance with aspects of the present inventive concepts. In the embodiment shown in FIG. 8A, the rotor system 100 comprises a rotor body 1 (or chassis), a sample region 40, a source of electromagnetic radiation 107, and a detector 108a. In alternative embodiments, the rotor system 100 comprises different numbers of sample regions 40, sources of electromagnetic radiation 107, and/or detectors 108. For example, in the present view, an additional detector 108b, associated with an additional source and sample region can be seen. Examples of the source of electromagnetic radiation 107 include, but are not limited to, a light-emitting diode, a laser diode, gas-discharge arc-lamps (e.g. deuterium arc lamp or a xenon arc lamp), and an incandescent source. Examples of operating wavelengths of electromagnetic radiation that are incident on the sample region 40 include, but are not limited to, 214 nm, 260 nm, 280 nm, and 395 nm.

In some embodiments, the rotor body 1 comprises one or more stacked subunits 30. In the embodiment shown in FIG. 8A, the rotor body 1 comprises five subunits 30a-e that are stacked and secured to each other. In alternative embodiments, the rotor body 1 comprises a different number of subunits 30 that are stacked and secured. In the embodiment shown in FIG. 8A, the top subunit is a source subunit 30a comprising at least one source of electromagnetic radiation 107. Below that, is a first window subunit 30b. In the embodiment shown in FIG. 8A the first window subunit 30b comprises a first window region 45a. In the embodiment shown, the source of electromagnetic radiation 107 extends into the first window region 45a. In FIG. 8A, the first window region 45a comprises a first window 110a at the lower surface. In some embodiments, the first window 110a is transparent to at least one wavelength emitted by the source of electromagnetic radiation 107. In some embodiments, the first and second window subunits 30b, 30d comprise a material that is fully transparent, partially transparent, or opaque to wavelengths of electromagnetic energy emitted by the source of electromagnetic radiation 107.

In the embodiment shown in FIG. 8A, a sample subunit 30c is positioned below the first window subunit 30b. The sample subunit 30c comprises at least one sample region 40. In this embodiment, the sample region 40 is positioned below the first window 110a of the first window region 45a of the first window subunit 30b.

In the embodiment shown in FIG. 8A, a second window subunit 30d is positioned below the sample subunit 30c. In this embodiment, the second window subunit 30d comprises the second window region 45b. In the embodiment shown, the first detector 108a extends into the second window region 45b. In FIG. 8A, the second window region 45b comprises a second window 110b at the upper surface. The second window 110b is positioned below the sample region 40. In some embodiments, the second window 110b is transparent to at least one wavelength emitted from the sample region 40.

In the embodiment shown in FIG. 8A, a detection subunit 30e is positioned below the second window subunit 30d. In this embodiment, the detection subunit 30e comprises the first detector 108a and the second detector 108b. In alternative embodiments, the detection subunit 30e comprises a different number of detectors.

In some embodiments, one or more of the one or more subunits 30 comprise an opening 31 constructed and arranged to allow for the insertion of a central projection (not shown in FIG. 8A). In some embodiments, the opening 31 is centered about an axis of rotation 103. In alternative embodiments, the opening 31 and shaft are off-center relative to the axis of rotation 103. In some embodiments, the rotor body 1 is symmetrical about the axis of rotation 103.

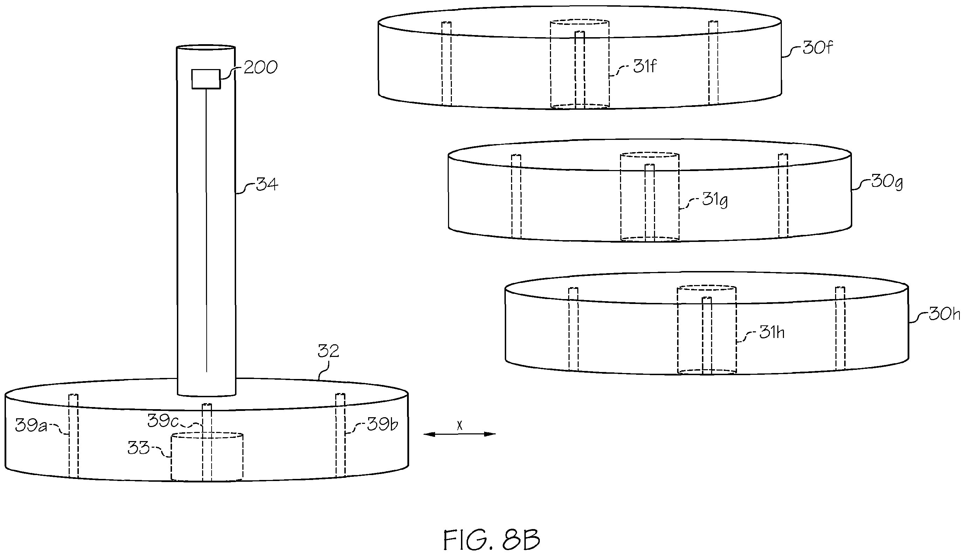

FIG. 8B is an exploded perspective view of an embodiment with four subunits, in accordance with aspects of the present inventive concepts. In this embodiment, a base subunit 32 comprises an acceptance hole 33, the acceptance hole 33 being constructed and arranged to couple with a drive shaft (not shown) from a centrifuge. In different embodiments, the acceptance hole 33 is constructed and arranged to couple with different commercially available centrifuges. In some embodiments, the base subunit 32 comprises one or more bolt holes 39. In the embodiment shown in FIG. 8B, the base subunit 32 comprises three bolt holes 39a-c, and different embodiments can comprise a different number of bolt holes.

In some embodiments, the base subunit 32 comprises a central projection 34. The central projection 34 extends in a vertical direction transverse to a horizontal direction of extension x of the base subunit 32. In some embodiments, the central projection 34 comprises a material of high strength and is configured to absorb and distribute the centrifugal force of the centrifuge's drive shaft. In some embodiments, the central projection 34 comprises a transmitter 200, the transmitter 200 being constructed and arranged to transmit information from the rotor system 100 to an external location. In some embodiments, the transmitter 200 comprises a broadcast antenna. In some embodiments, the transmitter 200 transmits a wireless electromagnetic signal.

In FIG. 8B, three additional subunits 30 f-h are illustrated. Each subunit comprises multiple bolt holes 39 and an opening 31. The opening is constructed and arranged to couple to the central projection 34 of the base subunit 32.

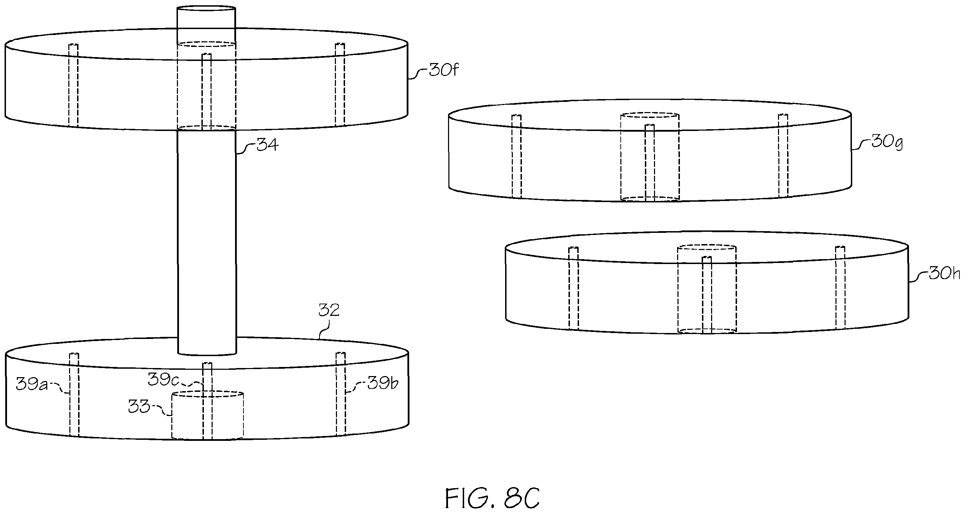

FIG. 8C is an exploded perspective view of an embodiment with four subunits, in accordance with aspects of the present inventive concepts. FIG. 8C illustrates the embodiment of FIG. 8B, with one of the subunits 30 coupled to the base subunit 32. The central projection 34 passes through the opening 31.

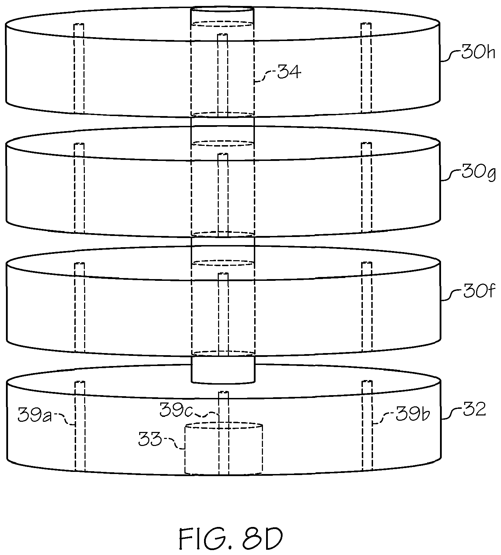

FIG. 8D is an exploded perspective view of an embodiment with four subunits, in accordance with aspects of the present inventive concepts. FIG. 8D illustrates the embodiment of FIG. 8B, with three of the subunits 30 coupled to the base subunit 32. The central projection 34 passes through the central opening of each additional subunit. The bolt holes 39 of each subunit are aligned such that a bolt could be inserted from the top or the bottom and passes through one of the bolt holes and be secured on the opposing side.

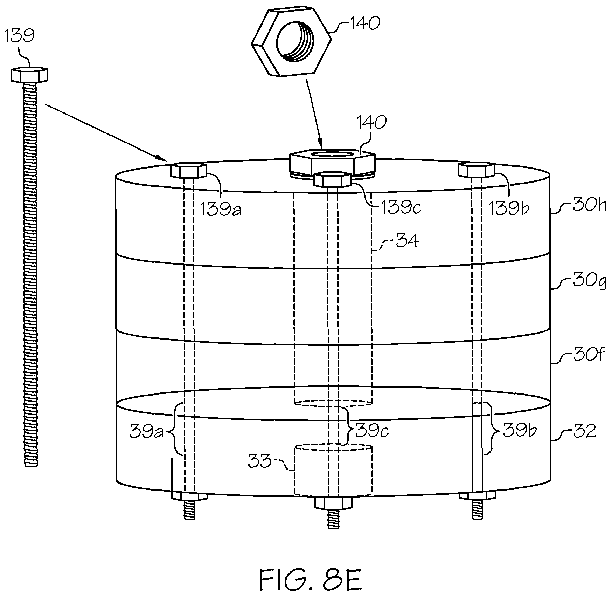

FIG. 8E is a perspective view of an embodiment with four subunits assembled, in accordance with aspects of the present inventive concepts. FIG. 8E illustrates bolts 139a-c positioned in the bolt holes 39a-c, allowing for air-tight, liquid-tight, rigid mating between subunits. In this embodiment, a central nut 140 secures the subunits 32, 30f-h at the central projection 34. In alternative embodiments, there may be threaded connections between the outer edges of each subunit 32, 30f-h for securing the subunits to each other. In other embodiments, the subunits may be secured to each other using other suitable securing mechanisms.

FIG. 8F is a perspective view of an embodiment of a subunit 30z comprising a sealing gasket 35, in accordance with aspects of the present inventive concepts. In some embodiments, a sealing gasket 35 is positioned at a groove 35a along the outer periphery of at least one subunit, facilitating an air-tight and liquid-tight junction between that subunit and a neighboring subunit.

In some embodiments, one or more of the one or more subunits 30 comprise one or more sample regions 40. The embodiment shown in FIG. 8A illustrates one sample region 40, but alternative embodiments comprise a different number of sample regions.

In some embodiments, one or more of the one or more subunits 30 comprise one or more window regions 45. In FIG. 8A, six window regions are illustrated: two on the right 45a, 45b, two on the left 45c, 45d, and two in the background 45e, 45f. In FIG. 8A, window regions 45a, 45b, and sample region 40 are sectioned.

In some embodiments, one or more of the one or more sample regions 40 comprises a sector shape. Specifically, a sector-shaped geometry is defined as the volume element of a cylindrical segment having the cross-sectional area of a circular sector (an area enclosed by two radii of a large circle, the bounding arc of the larger circle between the radii, and an arc of a smaller concentric circle of lesser radius). In addition, a sector-shaped geometry comprises at least two boundaries that coincide with radial lines that emanate outward from the axis of rotation. , the radial direction being defined as the direction that extends radially outward from the axis of rotation 103.

In some embodiments, the rotor system 100 comprises one or more functional components. The embodiment shown in FIG. 8A comprises three functional components: a source of electromagnetic radiation 107, a first detector 108a, and a second detector 108b. In the embodiment illustrated in FIG. 8A, the source of electromagnetic radiation 107 is arranged such that it is optically coupled with the first detector 108a. Alternative embodiments include additional functional elements including, but not limited to, integrated circuit boards, additional sources of electromagnetic radiation 107, and/or additional detectors 108. In the embodiment shown in FIG. 8A, the functional components are located within window regions 45a, 45b. In alternative embodiments, one or more functional components are positioned outside of window regions 45.

FIG. 8G is a perspective view of an embodiment of a subunit 30k including a functional element 149, in accordance with aspects of the present inventive concepts. Alternative embodiments comprise a plurality of functional components, including, but not limited to, a battery, memory, central processing unit, and wiring interconnects and/or plugs with neighboring subunits. In various embodiments, the functional element 149 can be mounted to the subunit 30k. In other embodiments, the functional element 149 can be embedded in the body of the subunit 30k.

FIG. 8G1 is a close-up perspective view of an embodiment of a functional element 149 and the associated connector 151, in accordance with embodiments of the present inventive concepts. In the embodiment shown in FIG. 8G1, the functional element 149 comprises a circuit board. In the embodiment shown in FIG. 8G1 the connector 151 comprises a ribbon cable. In alternative embodiments, the connector 151 comprises connections including, but not limited to USB connections, micro-USB connection, VGA connections, D-type connections, or other mechanisms suitable for transferring power and/or information.

In alternative embodiments, alternative geometries can be employed such that electromagnetic radiation can be guided in and out the sample region by various optics, for example light pipes, mirrors, fiber optics, focusing lenses, or other suitable optical devices.

In the embodiment shown in FIG. 8A, the sample region 40 comprises a sector shape. In some embodiments, the sample region 40 is constructed and arranged to contain a liquid. In some embodiments, the sample region 40 is constructed and arranged to contain a solid. In some embodiments, the sample region 40 is constructed and arranged to contain a gas.

FIG. 8H1 - 8H5 illustrate a method of assembling a rotor system 100, in accordance with embodiments of the present inventive concepts. In some embodiments, the rotor system 100 is assembled from the bottom up. For example, for assembling some embodiments, one subunit 30j comprising a sample region 40 is positioned above a second subunit 30i, as shown in FIG. 8H1. In this embodiment, the sample region 40 comprises sector-shaped side walls, and is open at the top and bottom.

FIG. 8H2 is a perspective view of an embodiment of the two subunits 30 i-j of FIG. 8H1 coupled with an air-tight and liquid-tight junction, in accordance with aspects of the present inventive concepts. In this embodiment, a surface in the lower subunit 30i serves as a lower surface for the sample region 40.

FIG. 8H3 is a perspective view of an embodiment of a sample being added to the sample region 40, in accordance with aspects of the present inventive concepts.

FIG. 8H4 is a perspective view of an embodiment of an additional subunit 30k being added above the sample region 40, in accordance with aspect of the present inventive concepts. In this embodiment, the top subunit 30k is coupled to another subunit 30j with an air-tight, liquid-tight seal. A surface in the top subunit 30k serves as an upper surface of the sample region 40.

FIG. 8H5 is a perspective view of an embodiment of the three subunits coupled together, in accordance with aspects of the present inventive concepts.

Alternative embodiments comprise a different number of subunits. For example, in assembling an embodiment such as the one illustrated in FIG. 8A, first the detector subunit 30e is positioned, then the second window subunit 30d, then the sample subunit 30c. In the embodiment shown in FIG. 8A the sample region 40 comprises side walls in a sector shape. In this embodiment, the top and bottom of the sample region 40 are open, however because the sample region 40 is positioned directly above the second window 110b, the second window 110b serves as a lower boundary for the sample region 40.

In some embodiments, the first window subunit 30b is positioned above the sample subunit 30c. In some embodiments, a sample is inserted into the sample region 40 before the first window subunit 30b is positioned above the sample subunit 30c. In such cases, the first window 110a is positioned directly above the sample region 40 and the first window 110a serves as a boundary for the sample region 40. In some embodiments, the source subunit 30a is positioned above the first window subunit 30b.

In some embodiments, the first window subunit 30b is positioned above the sample subunit 30c. In some embodiments, a sample is inserted into the sample region 40 before the first window subunit 30b is positioned above the sample subunit 30c. In such cases, the first window 110a is positioned directly above the sample region 40 and the first window 110a serves as a boundary for the sample region 40. In some embodiments, the source subunit 30a is positioned above the first window subunit 30b.

In some embodiments the sample region 40 comprises a sample chamber 106 that is constructed and arranged to be positioned in the sample region 40. In such embodiments, the sample chamber 106 comprises a top window that is transparent to one or more wavelengths in the electromagnetic spectrum. In some embodiments, the sample chamber 106 comprises a bottom window that is transparent to one or more wavelengths in the electromagnetic spectrum. In some embodiments, the top window of the sample chamber 106 is transparent to one or more wavelengths emitted by the source of electromagnetic radiation 107. In some embodiments, the bottom window is transparent to one or more wavelengths emitted from the sample region 40. In some embodiments, the sample chamber 106 comprises a key-way 118 such that it is coupled to the sample subunit. The key-way 118 can be constructed and arranged to hold the sample chamber 106 in position.

In some embodiments the sample chamber 106 is constructed and arranged to be removable from the sample region 40. In the embodiment shown in FIG. 8A, the sample chamber 106 comprises a sector shape. In some embodiments, the sample chamber 106 is constructed and arranged to contain a liquid. In some embodiments, the sample chamber 106 is constructed and arranged to contain a solid. In some embodiments, the sample chamber 106 is constructed and arranged to contain a gas.

In the embodiment of FIG. 8A, the sample chamber 106, the source of electromagnetic radiation 107, and the first detector 108a are all shown in-register along an axis that is parallel to the axis of rotation 103. In this embodiment, the first window region 45a comprises a first window 110a above the sample region 40 and that window 110a is transparent to one or more wavelengths emitted by the source of electromagnetic radiation 107. In this embodiment, the second window region 45b comprises a second window 110b below the sample region 40 and that window 110b is transparent to one or more wavelengths designed to reach the first detector 108a.

In some embodiments, the windows 110a, 110b at each window region 45a, 45b and the windows at the sample chamber 106 may all pass substantially similar wavelengths of electromagnetic radiation. In such cases, the source of electromagnetic radiation 107 may be aligned with the first detector 108a. The electromagnetic radiation may pass through the window 110a at the first window layer 45a, pass through the window at the sample chamber 106, and interact with the sample. The scattered electromagnetic radiation may then pass through the lower window in the sample chamber 106, the window 110b at the second window layer 45b, and then reach the detector 108a.

In some embodiments, different windows transmit different wavelengths of electromagnetic radiation. For example, the electromagnetic radiation emitted from the source of electromagnetic radiation 107 may need to be filtered before propagating to the sample region 40. In such cases, the first window1 10a at the first window layer 45a and/or the top window at the sample chamber 106 may perform such filtering.

In some embodiments, the wavelengths of electromagnetic radiation to be detected may not match the wavelengths of electromagnetic radiation emitted from the source of electromagnetic radiation 107 (e.g. fluorescence imaging, Raman imaging, etc.). In such cases, the window 110b at the second window layer 45b and/or the bottom window at the sample chamber 106 may perform the necessary filtering.

In the embodiment shown in FIG. 8A, the source of electromagnetic radiation 107 is positioned above the sample chamber 106 and the detector 108a is positioned below the sample chamber 106. In alternative embodiments, the source of electromagnetic radiation 107 is positioned below the sample chamber 106 and the detector 108a is positioned above the sample chamber 106. In alternative embodiments, both the source of electromagnetic radiation 107 and the detector 108a are positioned below the sample chamber 106. In alternative embodiments, both the source of electromagnetic radiation 107 and the detector 108a are positioned above the sample chamber 106.

In some embodiments, the rotor system 100 comprises other components, such as additional integrated circuits, memory units, transmitter/receivers, and sensors. Such components may likewise be located on the various subunits 30 and may be positioned with respect to the axis of rotation 103 so as to provide a symmetric weight distribution for the resulting rotor.

FIG. 9 is a perspective sectional view of an embodiment of a rotor system 100, in accordance with aspects of the present inventive concepts. In the embodiment shown in FIG. 9, the rotor system 100 comprises a central shaft 36, fitted to the assembled rotor 1. In the embodiment shown, the rotor system 100 also comprises a bottom flange 37 and a top nut 38, which both serve to tension the stacked layers 30 of the rotor body 1 together as a rigid unit. In some embodiments, the lower end of the shaft 36 is fixed to a drive shaft or spindle of a centrifuge (not shown). In some embodiments, the lower end of shaft 36 is configured to be adaptable to accept a plurality of drive shaft or spindle dimensions that is common to many commercial centrifuges.

FIG. 10 is a perspective sectional view of an embodiment of a rotor system 100, in accordance with aspects of the present inventive concepts. FIG. 10 illustrates the rotor system 100 of FIG. 9 in which the source of electromagnetic radiation 107 directs electromagnetic radiation 50 through the sample chamber 106 and onto the detector 108a. In the embodiment shown in FIG. 10, the beam of electromagnetic radiation 50 diverges as it moves towards the detector 108a. In alternative embodiments, the source of electromagnetic radiation 107 emits electromagnetic radiation 50 with a different divergence. In alternative embodiments, the rotor system 100 comprises at least one collimator that collimates the beam of electromagnetic radiation 50 as it traverses the sample region 40. In some embodiments, the electromagnetic radiation 50 is directed over the entire sample region 40. In alternative embodiments, the electromagnetic radiation 50 is directed over the entire sample region 40 and is collimated as it is directed over the entire sample region 40.

FIG. 11A is an exploded perspective view of an embodiment of a rotor system 100 in accordance with aspects of the present inventive concepts. The layers or subunits are shown separated, as would be the case prior to assembly of the unit. In the embodiment shown in FIG. 11A, a sample subunit 30c comprises at least one removable sample chamber 106 at the sample region 40. In this embodiment, the first window region 45a, the second window region 45b, and the sample region 40 comprise a sector-shaped geometry. In this embodiment, the sample chamber 106 also comprises a sector-shaped geometry. In some embodiments, the first window region 45a does not comprise a sector-shaped geometry. In some embodiments, the second window region 45b does not comprise a sector-shaped geometry.

In the embodiment shown in FIG. 11A, the rotor system 100 comprises two window layers 30b, 30d. One window layer 30b is positioned directly above the sample subunit 30c. The other window layer 30d is positioned directly below the sample subunit 30c. The first window layer 30b comprises a window region 45a positioned above the sample chamber 106. In the embodiment shown in FIG. 11A, the first window 110a of the window region 45a, which is above the sample chamber 106, is transparent to at least a portion of the electromagnetic spectrum. In the embodiment shown in FIG. 11A, the second window 110b of the second window region 45b, which is below the sample chamber 106, is transparent to at least a portion of the electromagnetic spectrum. In some embodiments, directly above and below the sample region 40 are window layers 30b and 30d, which comprise transparent windows 110a, 110b that allow for the transmittance of electromagnetic radiation through the sample region 40. In the embodiment shown in FIG. 11A, a source subunit 30a is positioned above the first window subunit 30b. In the embodiment shown in FIG. 11A, a detection subunit 30e is below the second window subunit 30d. Circuit components representing the source of electromagnetic radiation 107 and the detector 108a are illustrated in FIG. 11A. In some embodiments, other functional circuit components may also be provided in the rotor 1, having functionality including but not limited to data storage, computational memory, thermostatic unit(s), electronic sensors, and transmitter/receivers.

FIG. 11B is a top view of an embodiment of a sample subunit 30m and a corresponding source subunit 30l, in accordance with aspects of the present inventive concepts. In this embodiment, each sector-shaped sample region 40 in the source subunit is aligned in the vertical direction with a corresponding one or more illumination elements 70 at the source subunit 30l.

FIG. 11C is a top view of an embodiment of a source subunit 30l and FIG 11C1 is a close-up perspective view of an embodiment of one or more illumination elements 70 at a position at the source subunit 30l, in accordance with aspects of the present inventive concepts. In this embodiment, at one position, the one or more illumination elements 70 comprises a source of electromagnetic radiation 107, an optical filter 72, an optical collimator 74, and a window 76. In some embodiments, the source of electromagnetic radiation 107, the optical filter 72, the optical collimator 74, and the window 76 are stacked. In alternative embodiments, the source subunit 30l comprises an optical diffuser.

FIG. 11D1 and FIG. 11D2 are perspective views of an embodiment of sources of electromagnetic radiation, in accordance with aspects of the present inventive concepts. In some embodiments, a source of electromagnetic radiation 107 is positioned above each sample region 40. In some embodiments, the source of electromagnetic radiation 107 comprises one or more light-emitting diodes. In alternative embodiments, there may be one or more sources of electromagnetic radiation 107 that direct incident electromagnetic radiation 50 to the sample region 40 via fiber optics, mirrors, waveguides, or other mechanisms suitable for conveying electromagnetic energy.

FIG. 11E is a top view of an embodiment of a sample subunit 30m and a corresponding detection subunit 30n, in accordance with aspects of the present inventive concepts. In this embodiment, each sector-shaped sample region 40 in the sample subunit 30m is aligned in the vertical direction with a corresponding one or more detection elements 80 at the detector subunit 30n.

FIG. 11F is a top view of an embodiment of a detection subunit 30n and FIG 11F1 is a close-up perspective view of an embodiment of one or more detection elements 80d, in accordance with aspects of the present inventive concepts. In this embodiment, at one position, the one or more detection elements 80d comprise a detector 108, a lens system 82, an optical filter 84, and a window 86. In alternative embodiments, the detection subunit 30n comprises additional optical elements. In some embodiments, the one or more detection elements 80d are arranged differently.

FIG. 11G1 is a perspective view of an embodiment of a rotor system 100 comprising a Schlieren image detection system, in accordance with aspects of the present inventive concepts. In this embodiment, the source subunit 30 comprises a window 76, and the detection subunit comprises a window 86. Electromagnetic radiation passes through the window 76 in the source subunit and into the sample region 40. The electromagnetic radiation from the sample region 40 passes through the window 86 on the detection subunit and passes through a focusing lens 92. The electromagnetic radiation is focused though an iris 94. The iris 94 can be positioned at the focal position of the focusing lens 92. In this embodiment, a Schlieren image is formed at the detector 108. In alternative embodiments, the iris 94 is replaced with a knife-edge or any suitable edge geometry capable of blocking a portion of the electromagnetic radiation.

FIG. 11G2 is a conceptual diagram of an embodiment of a rotor system 100 configured to perform Schlieren imaging, in accordance with aspects of the present inventive concepts. This embodiment is similar to the embodiment of FIG. 11G1. In this embodiment, temperature control elements 109 are positioned near the sample region 40. In this embodiment, the source of electromagnetic radiation 107, the detector 108, and the temperature control elements 109 are coupled to a CPU 10A, memory 10B, a battery 10C, a wireless transmitter 10D, and/or additional circuitry.

FIG. 11H1 is a perspective view of an embodiment of a rotor system 100 comprising a hyperspectral image detection system, in accordance with aspects of the present inventive concepts. In this embodiment, the source subunit comprises a window 76 and the detection subunit comprises a window 86. Electromagnetic radiation passes through the window 76 at the source subunit and into the sample region 40. The electromagnetic radiation from the sample region 40 passes through the window 86 at the detection subunit and passes through a lens system 82. The electromagnetic radiation then passes through a Fabry-Perot interferometer 96 before reaching the detector 108.

FIG. 11H2 is a conceptual diagram of an embodiment of a rotor system 100 configured to perform hyperspectral imaging, in accordance with aspects of the present inventive concepts. This embodiment is similar to the embodiment of FIG. 11H1. In this embodiment, temperature control elements 109 are positioned near the sample region 40. In this embodiment, the source of electromagnetic radiation 107, the detector 108, and the temperature control elements 109 are coupled to a CPU 10A, memory 10B, a battery 10C, a wireless transmitter 10D, and/or additional circuitry.

FIG. 11I is a conceptual diagram of an embodiment of a rotor system 100 with a filtered illumination source, in accordance with embodiments of the present inventive concepts. In this embodiment, electromagnetic radiation, from the source of electromagnetic radiation 107, passes through an optical diffuser 78 and a filter 75, such as a tunable Fabry-Perot filter, before passing through a collimator 74, then entering the sample region 40. The detector 108 collects electromagnetic radiation from the sample region 40. In this embodiment, temperature control elements 109 are positioned near the sample region 40. In this embodiment, the source of electromagnetic radiation 107, the detector 108, and the temperature control elements 109 are coupled to a CPU 10A, memory 10B, a battery 10C, a wireless transmitter 10D, and/or additional circuitry.

FIG. 11J is a perspective view of an embodiment of a central projection 34 comprising a source of electromagnetic radiation 107, in accordance with aspects of the present inventive concepts. In this embodiment, electromagnetic radiation 50 from the source of electromagnetic radiation 107 is directed through the central projection 34. In some embodiments, the central projection 34 comprises one or more mirrors 98. In some embodiments, the electromagnetic radiation 50 is directed to the one or more mirrors 98 and is then directed to one or more subunits 30. In some embodiments, the central projection 34 comprises one or more beam splitters 99. In some embodiments, the electromagnetic radiation 50 is directed to the one or more beam splitters 99 and is then directed to one or more subunits 30 and/or an additional mirror 98 and/or an additional beam splitter 99. In some embodiments, the source of electromagnetic radiation 107 in the central projection 34 emits electromagnetic radiation 50 of a higher intensity than a source of electromagnetic radiation 107 positioned in an individual subunit 30.

FIG. 12A is side view of an embodiment of a rotor system 100 in accordance with aspects of the present inventive concepts. FIG. 12A illustrates the assembled stacked layers (30a-e) fitted onto the shaft 36 and tensioned by the flange 37 and the nut 38. The embodiment shown in FIG. 12A comprises a plurality of sources of electromagnetic radiation 107 and a plurality of detectors 108. FIG. 12A illustrates that in alternative embodiments, a plurality of sample chambers 106 may be provided, each with corresponding sources of electromagnetic radiation 107 and detectors 108.

FIG. 12B1 is a perspective view of an embodiment of a subunit 30 comprising at least one sample region 40 and a temperature control system 700, in accordance with aspects of the present inventive concepts. In some embodiments, the temperature control system 700 comprises one or more temperature control elements 109, such as, but not limited to, a Peltier unit (FIG. 12B4). In some embodiments, one or more temperature control elements 109 neighbor one or more of the sample regions 40 in the rotor system 100. In some embodiments, there are fewer temperature control elements 109 than sample regions 40.

In some embodiments, the temperature control system 700 comprises an active pumping mechanism and one or more thermal channels 720. In some embodiments, the active pumping mechanism pumps a thermally conductive fluid 730 through the one or more thermal channels 720. The temperature control elements 109 heat or cool the thermally conductive fluid 730 and the fluid is pumped through the thermal channels 720. The thermal channels 720 are positioned near one or more sample regions 40 and the presence of the thermally conductive fluid 730 modifies the temperature in the sample region 40.

In some embodiments, the temperature control system 700 comprises one or more stirrers 710. In some embodiments, the one or more stirrers 710 help to move the thermally conductive fluid 730 through the thermal channels 720.

FIG. 12B2 is a close-up perspective view of an embodiment of a sample region 40 from FIG. 12B1, in accordance with aspects of the present inventive concepts. In the embodiments shown in FIG. 12B2, the temperature control system 700 comprises a temperature sensor 740 that is coupled to the sample region 40. The temperature sensor 740 measures the temperature at the sample region 40 and transmits that information to the temperature control system 700, via a wired or wireless connection. In some embodiments, the temperature control system 700 compares the measured temperature to a predetermined set point. In some embodiments, if the measured temperature deviates from the predetermined set point, the temperature control system 700 takes steps to reduce this difference. In some embodiments, the temperature control system 700 reduces the temperature difference by adjusting one or more temperature control elements 109 or by adjusting the flow of the thermally conductive fluid 730. In some embodiments, the temperature sensor 740 comprises an alarm that indicates when the temperature deviates by a certain amount from a predetermined set point. In the embodiment shown in FIG. 12B2, a thermal channel 720 neighbors the sample region 40 and is oriented along the axis of rotation 103.

FIG. 12B3 is a top view of an embodiment of a sample subunit 30 comprising a thermal channel 720, in accordance with aspects of the present inventive concepts. In this embodiment, the sample subunit 30 comprises four sample regions 40, symmetrically aligned about the rotor's axis of rotation 103. The thermal channel 720 extends symmetrically though the sample subunit (only the upper left quadrant is displayed). In this embodiment, the thermal channel 720 is oriented transverse to the axis of rotation 103.

FIG. 12B4 is a close-up perspective view of an embodiment of a temperature control element 109 and a stirrer 710, in accordance with aspects of the present inventive concepts.

FIG. 12C is a side view of an embodiment of a centrifugally-driven voltaic cell 800 constructed and arranged to provide electrical power to one or more subunits 30, in accordance with aspects of the present inventive concepts. In some embodiments, the cell 800 is configured in the form of a concentration cell and is oriented transverse relative to the axis of rotation 103 of the rotor. In some embodiments, each cell 800 comprises two electrodes (terminals) of the same material: an anode 810 and cathode 820, separated along the radial dimension by an electrolyte solution 830 containing the ionized atomic or molecular form of the two parent electrodes. In some embodiments, the electrodes 810, 820 are arranged in-line, such that they are at the same elevation. Each electrode 810, 820 interacts with the electrolyte solution 830 by gaining or losing material by chemical reaction (oxidation / reduction) at each electrode surface.

In addition to being connected via the electrolyte solution 830, a separate electrical wiring connection 840 is maintained between the two terminals 810, 820. In the figure, current is driven through the wiring, as shown, by an induced ion concentration gradient which occurs due to the centrifugal force exerted on the system. In some embodiments, unlike the conventional concentration-type voltaic cell, the local ionic concentration at the vicinity of each electrode is prevented from being equalized as long as sufficient centrifugal force is applied, and therefore will run under centrifugation as long as solid electrode material is available at each terminal. In some embodiments, a multitude of cells of the type shown here may be interconnected in series and/or in parallel, to form a battery-like configuration which may supply power on board the rotating device.

FIG. 12D is a side view of an embodiment of a voltaic cell 850 constructed and arranged to be driven by beta particle emission from radio-isotope decay, to provide electrical power to one or more subunits 30, in accordance with embodiments of the present inventive concepts. In some embodiments, the cell 850 is configured in the form of a stack of alternating materials, or a plurality of such stacks, with boundaries between the materials, oriented transverse relative to the axis of rotation 103 of the rotor.

In some embodiments, each cell 850 comprises a topmost layer which comprises a material that emits beta particles by radio-isotopic decay 860. In some embodiments, immediately below the beta emitting layer 860 is a layer comprising n-type semiconductor material 870, which releases electrons, as donors, upon irradiation with high energy beta particles. In some embodiments, immediately below the n-type material is a layer comprising p-type semiconductor material 880, forming a p-n semiconductor junction. In some embodiments, the layer comprising p-type semiconductor material 880 is positioned above a base 890.

Irradiation of the n-type layer 870 above the p-n junction drives a current through terminals 875, 885 attached at the junctions above and below the p-n junction, as shown, which may drive electrical work when connected to a load.

It is noted that the mounting systems described herein at least in connection with FIGS. 8A-12D and the corresponding text in relation to the rotor system 100 are equally applicable to the integrated cell system 500 described at least at FIGS. 13-17 and the corresponding text. For the sake of brevity, an additional description of such systems is not repeated in connection with the description of the integrated cell system 500.

It is noted that the subunit systems described herein at least in connection with FIGS. 8A-12D and the corresponding text in relation to the rotor system 100 are equally applicable to the integrated cell system 500 described at least at FIGS. 13-17 and the corresponding text. For the sake of brevity, an additional description of such systems is not repeated in connection with the description of the integrated cell system 500.

It is noted that the window systems described herein at least in connection with FIGS. 8A-12D and the corresponding text in relation to the rotor system 100 are equally applicable to the integrated cell system 500 described at least at FIGS. 13-17 and the corresponding text. For the sake of brevity, an additional description of such systems is not repeated in connection with the description of the integrated cell system 500.

It is noted that the battery systems described herein at least in connection with FIGS. 8A-12D and the corresponding text in relation to the rotor system 100 are equally applicable to the integrated cell system 500 described at least at FIGS. 13-17 and the corresponding text. For the sake of brevity, an additional description of such systems is not repeated in connection with the description of the integrated cell system 500.

It is noted that the optical systems described herein at least in connection with FIGS. 8A-12D and the corresponding text in relation to the rotor system 100 are equally applicable to the integrated cell system 500 described at least at FIGS. 13-17 and the corresponding text. For the sake of brevity, an additional description of such systems is not repeated in connection with the description of the integrated cell system 500.

It is noted that the temperature control systems described herein at least in connection with FIGS. 8A-12D and the corresponding text in relation to the rotor system 100 are equally applicable to the integrated cell system 500 described at least at FIGS. 13-17 and the corresponding text. For the sake of brevity, an additional description of such systems is not repeated in connection with the description of the integrated cell system 500.

FIG. 13 is a perspective sectional view of an embodiment of an integrated cell device (Smart Cell) 500, also referred to as an interrogation cell, in accordance with aspects of the present inventive concepts. In the embodiment shown in FIG. 13, the integrated cell device 500 comprises a cell housing 516, or outer sleeve. In some embodiments, fitted inside the cell housing 516 are one or more stacked subunits 530. The subunits 530 themselves may separately house different functional components, and the subunits 530 are stacked and secured. In some embodiments, the stacked subunits 530 are bounded on the top by a tensioning screw or cap 517. In some embodiments, the tensioning screw or cap 517 fits inside the cell housing 516 and provides a force to secure all subunits 530 together such that the integrated cell device 500 acts as a rigid unit. The embodiment shown in FIG. 13, a sector-shaped, removeable sample chamber 506 is positioned in a sample region 540 at the sample subunit 530c. Directly above the sample subunit 530c is a first window subunit 530b. The first window subunit 530b comprises a first window region 545a. The first window region 545a comprises at least one window 510a positioned above the sample region 540.

Directly below the sample subunit 530c is a second window subunit 530d. The second window subunit 530b comprises a second window region 545b. The second window region 545b comprises at least one window 510b positioned below the sample region 540. In this embodiment, each window subunit 530b, d comprises a transparent window 510a, b that allows for the transmittance of electromagnetic radiation through the sample region 540. The subunits 530 may comprise other functional components, which may be components of integrated circuits on printed circuit boards, including, but not limited to, a source subunit 530a and a detection subunit 530e. In the embodiment shown in FIG. 13, the sample region 540 comprises a removable sample chamber 506. In some embodiments, the characteristics of the sample chamber 506 are similar to those characteristics described in connection with the sample chamber 106 of the rotor system 100. In some embodiments, the sample region 540 is constructed and arranged to contain a liquid. In some embodiments, the sample region 540 is constructed and arranged to contain a solid. In some embodiments, the sample region 540 is constructed and arranged to contain a gas.

In some embodiments, the source subunit 530a comprises a source of electromagnetic radiation 524. Examples of the source of electromagnetic radiation 524 include, but are not limited to, a light-emitting diode, a laser diode, gas-discharge arc-lamps (e.g. deuterium arc lamp or a xenon arc lamp), and an incandescent source. In some embodiments, the detection layer 530e comprises a detector 525. In some embodiments, the detector 525 is coupled to a circuit. In some embodiments, the detector comprises a detector array.

In the embodiment of FIG. 13, the sample chamber 506, the source of electromagnetic radiation 524, and the detector 525 are all shown in-register along an axis that is parallel to the elongated axis of the cell housing 516. In this embodiment, window region 545a comprises a window 510a above the sample region 540 and that window 510a is transparent to one or more wavelengths emitted by the source of electromagnetic radiation 524. In this embodiment, window region 545b comprises a window 510b below the sample region 540 and that window 510b is transparent to one or more wavelengths designed to reach the detector 525.

In some embodiments, the windows 510a,b in each window region 545a, 545b and the windows on the sample chamber 506 may all pass substantially similar wavelengths of electromagnetic radiation. In such cases, the source of electromagnetic radiation 524 may be aligned with the first detector 525. The electromagnetic radiation may pass through the window 510a at the first window layer 545a, pass through the window at the sample chamber 506, and interact with the sample. The scattered electromagnetic radiation then passes through the lower window in the sample chamber 506, the window 510b at the second window layer 545b, and then reaches the detector 525.

In some embodiments, different windows transmit different wavelengths of electromagnetic radiation. For example, the electromagnetic radiation emitted from the source of electromagnetic radiation 524 may need to be filtered before propagating to the sample region 540. In such cases, the window 510a at the first window layer 545a and/or the top window at the sample chamber 506 may perform such filtering.

In some embodiments, the wavelengths of electromagnetic radiation to be detected may not match the wavelengths of electromagnetic radiation emitted from the source of electromagnetic radiation 524 (e.g. fluorescence imaging, Raman imaging, etc.). In such cases, the window 510b at the second window layer 545b and/or the bottom window at the sample chamber 506 may perform the necessary filtering.

In the embodiment shown in FIG. 13, the source of electromagnetic radiation 517 is positioned above the sample region 540 and the detector 525 is positioned below the sample region 540. In alternative embodiments, the source of electromagnetic radiation 524 is positioned below the sample region 540 and the detector 525 is positioned above the sample region 540. In alternative embodiments, both the source of electromagnetic radiation 524 and the detector 525 are positioned below the sample region 540. In alternative embodiments, both the source of electromagnetic radiation 524 and the detector 525 are positioned above the sample region 540.

In alternative embodiments, other components, such as additional integrated circuits, memory units, transmitter/receivers, and sensors may likewise be located on one or more layers. In some embodiments, additional components are symmetrically positioned with respect to the axis of rotation.

In the embodiment shown in FIG, 13, the cell device 500 is shown with electromagnetic radiation 26 directed through the sample chamber 506 and incident at the detector 525, which is situated below the sample chamber 506. In the embodiment shown in FIG. 13, the electromagnetic radiation 50 diverges as it moves towards the detector 525.

In alternative embodiments, the source of electromagnetic radiation 524 emits electromagnetic radiation with a different divergence. In alternative embodiments, the cell 500 comprises at least one collimator that collimates the beam of electromagnetic radiation 26 as it traverses the sample region 540. In some embodiments, the electromagnetic radiation 26 is directed over the entire sample region 540. In alternative embodiments, the electromagnetic radiation 26 is directed over the entire sample region 540 and is collimated as it is directed over the entire sample region 540.

Although the electromagnetic radiation 26, illustrated in FIG. 13 is depicted as originating from a point source, other configurations of the electromagnetic radiation are attainable in certain embodiments, to allow for uniform, collimated electromagnetic radiation to be directed over the entire length of the sample region 540, and subsequent complete detection of the transmitted electromagnetic footprint by the detector 525.

FIG. 14 is an exploded sectional view of an embodiment of an integrated cell device 500, in accordance with aspects of the present inventive concepts. In the embodiment shown in FIG. 14, the subunits are shown separated, as would be the case prior to assembly of the unit, in preparation for use. The sample subunit 530c is constructed and arranged to house the sample chamber 506. In some embodiments, the sample region 540 comprises a sector-shaped geometry as depicted in FIG. 14. Also illustrated in the sample subunit 530c is the sample chamber 506, which also may comprise a sector-shaped geometry. Directly above and below the sample subunit 530c are the first window subunit 530b and the second window subunit 530d, which have transparent windows that allow for the transmittance of electromagnetic radiation through the sample region 540. In the embodiment shown in FIG. 14, above the first window subunit 530b is shown the source subunit 530a. In this embodiment, directly below the second window subunit 530d is the detection subunit 530e. Circuit components representing, the source of electromagnetic radiation 524 and the detector units 525 are illustrated in FIG. 14, although in certain embodiments other functional circuit components may also be provided having functionality including but not limited to data storage, computational memory, thermostatic unit(s), electronic sensors, and transmitter/receivers.

FIG. 15 is a perspective sectional view of an embodiment of an assembled integrated cell device 500, in accordance with embodiments of the present inventive concepts. In the embodiment shown in FIG. 15, the cell device 500 comprises an outer sleeve 516, top cap 517, and internal components as described previously. In some embodiments, the cell device 500 is dimensioned to fit into rotor form factors that are commercially available. In some embodiments, the cell device 500, is a one-for-one replacement for a conventional cell.

FIG. 16 is a perspective sectional view of an embodiment of an assembled cell device 500, in accordance with aspects of the present inventive concepts. In this embodiment, the cell device 500 comprises an outer sleeve 16, top cap 17, and internal components as described previously. In FIG. 16, electromagnetic radiation 26 is directed through the sample chamber 506 and onto the detector unit 525, situated below the sample chamber 506. Although the electromagnetic radiation 26, illustrated in FIG. 16 is depicted as originating from a point source, other configurations of the electromagnetic radiation are attainable in certain embodiments, to allow for uniform, collimated electromagnetic radiation to be directed over the entire length of the sample region 540, and subsequent complete detection of the transmitted electromagnetic footprint by the detector 525.

FIG. 17 is a perspective view of an embodiment of an assembled cell device shown fitted inside rotor 127, in accordance with aspects of the present inventive concepts. FIG. 17 illustrates the 1:1 correspondence of a cell device 500 with a rotor hole, as makes clear that multiple cell devices 500 may separately be fitted into distinct rotor holes of a given rotor 127.

The above-described embodiments should be understood to serve only as illustrative examples; and equivalents and modifications not described above may also be employed without departing from the scope of the specification, which is defined in the accompanying claims.

## Claims

1. An interrogation cell (500), comprising:
a source of electromagnetic radiation (524) at a first position, the source of electromagnetic radiation configured to emit electromagnetic radiation (524) at one or more wavelengths;
a sample region (540); and
a detector (525) at a second position, the detector configured to receive electromagnetic radiation that traverses at least a portion of the sample region,
**characterized in that** the interrogation cell (500) is dimensioned for positioning in a rotor cavity of a centrifuge rotor (127).

2. The interrogation cell (500) of claim 1, wherein the sample region (540) comprises a sector shape wherein the sample region comprise a sample chamber (506) and wherein the sample chamber (506) comprise a removable liner.

3. The interrogation cell (500) of claim 1, wherein system is configured to provide spectroscopic information of the sample region (540) based on the stored information collected by the detector (525) and wherein the system is configured to provide at least one type of spectroscopic information from a group consisting of: hyperspectral image data, Schlieren images, fluorescence images, or quantitative fluorescence emission data comprising a spatial arrangement of the sample region (540), or combinations thereof.

4. The interrogation cell (500) of claim 3, further comprising at least one component optically coupled with the source of electromagnetic radiation (524) and the detector (525), the at least one component being at least one component selected from a group consisting of: at least one Fabry-Perot interferometer, one or more optical filters, one or more optical lenses, one or more mirrors, one or more optical diffusers, or one or more optical collimators, or combinations thereof.

5. The interrogation cell (500) of claim 4, wherein one or more of the one or more optical collimators comprises at least one self-collimating photonic crystal.

6. The interrogation cell (500) of claim 4, wherein one or more of the one or more optical collimators comprises at least one micro-Fresnel lens.

7. The interrogation cell (500) of claim 3, further comprising a beamsplitter system constructed and arranged to direct electromagnetic radiation (524) from the source of electromagnetic radiation (524) to a plurality of locations and wherein the beamsplitter comprises at least one component from a group consisting of: at least one mirror, at least one filter, at least one lens, or combinations thereof.

8. The interrogation cell (500) of claim 1, further comprising a temperature control system (109) configured to modify a temperature of the sample region (540).

9. The interrogation cell (500) of claim 8, wherein the temperature control system is configured to maintain the temperature of the sample region (540), wherein maintaining the temperature comprises heating the sample region (540), and wherein maintaining the temperature comprises cooling the sample region (540).

10. The interrogation cell (500) of claim 8, wherein the temperature control system (109) comprises a temperature sensor (740) that comprises at least one item from a group consisting of: at least one thermocouple, at least one optical sensor, at least one infrared sensor, or combinations thereof.

11. The interrogation cell (500) of claim 8, wherein the temperature control system (109) comprises a thermoelectric or Peltier device.

12. The interrogation cell (500) of claim 1, wherein the interrogation cell (500) comprises a power source (10C), and wherein the power source comprises at least one battery.

13. The interrogation cell (500) of claim 1, wherein the power source comprises a recharging mechanism constructed and arranged to convert rotational energy of the interrogation cell (500) into electrical current.

14. The interrogation cell (500) of claim 13, wherein the power source comprises at least one voltaic cell (850), wherein the at least one voltaic cell comprises two electrodes separated by an electrolyte solution, in the form of a concentration cell, and wherein the current is driven by an electrolyte concentration difference in the electrolyte solution that is induced by centrifugation.

15. The interrogation cell (500) of claim 1, wherein the interrogation cell (500) comprises a power source, wherein the power source comprises at least one battery, and wherein the at least one battery is at least one voltaic cell (850) and is driven by a radio-isotopic decay (860).

## Patentansprüche

1. Eine Abfragezelle (500), umfassend:
eine Quelle elektromagnetischer Strahlung (524) an einer ersten Position, wobei die Quelle elektromagnetischer Strahlung so konfiguriert ist, dass sie elektromagnetische Strahlung (524) mit einer oder mehreren Wellenlängen ausstrahlt;
einen Probenbereich (540); und
einen Detektor (525) an einer zweiten Position, wobei der Detektor so konfiguriert ist, dass er elektromagnetische Strahlung empfängt, die mindestens einen Teil des Probenbereichs durchquert,
**dadurch gekennzeichnet, dass** die Abfragezelle (500) zur Positionierung in einem Rotorhohlraum eines Zentrifugenrotors (127) dimensioniert ist.

2. Abfragezelle (500) nach Anspruch 1, wobei der Probenbereich (540) eine Sektorform umfasst, wobei der Probenbereich eine Probenkammer (506) umfasst und wobei die Probenkammer (506) eine entfernbare Auskleidung umfasst.

3. Abfragezelle (500) nach Anspruch 1, wobei das System so konfiguriert ist, dass es spektroskopische Informationen des Probenbereichs (540) auf der Grundlage der vom Detektor (525) gesammelten gespeicherten Informationen bereitstellt, und wobei das System so konfiguriert ist, dass es mindestens eine Art von spektroskopischen Informationen aus einer Gruppe bereitstellt, die besteht aus: Hyperspektralbilddaten, Schlierenbildern, Fluoreszenzbildern oder quantitativen Fluoreszenzemissionsdaten, die eine räumliche Anordnung des Probenbereichs (540), oder Kombinationen davon.

4. Abfragezelle (500) nach Anspruch 3 umfasst ferner mindestens eine Komponente, die optisch mit der Quelle elektromagnetischer Strahlung (524) und dem Detektor (525) gekoppelt ist, wobei es sich bei der mindestens einen Komponente um mindestens eine Komponente handelt, die aus einer Gruppe ausgewählt ist, die besteht aus: mindestens einem Fabry-Perot-Interferometer, einem oder mehreren optischen Filtern, einer oder mehreren optischen Linsen, einem oder mehreren Spiegeln, einem oder mehreren optischen Diffusoren oder einem oder mehreren optischen Kollimatoren oder Kombinationen davon.

5. Abfragezelle (500) nach Anspruch 4, wobei einer oder mehrere der einen oder mehreren optischen Kollimatoren mindestens einen selbstkollimierenden photonischen Kristall umfassen.

6. Abfragezelle (500) nach Anspruch 4, wobei einer oder mehrere der einen oder mehreren optischen Kollimatoren mindestens eine Mikro-Fresnel-Linse umfassen.

7. Abfragezelle (500) nach Anspruch 3 umfasst ferner ein Strahlenteilersystem, das so konstruiert und angeordnet ist, dass es elektromagnetische Strahlung (524) von der Quelle elektromagnetischer Strahlung (524) auf eine Vielzahl von Orten lenkt, und wobei der Strahlenteiler mindestens eine Komponente aus einer Gruppe umfasst, die besteht aus: mindestens einem Spiegel, mindestens einem Filter, mindestens einer Linse oder Kombinationen davon.

8. Abfragezelle (500) nach Anspruch 1 umfasst ferner ein Temperatursteuerungssystem (109), das so konfiguriert ist, dass es die Temperatur des Probenbereichs (540) modifiziert.

9. Abfragezelle (500) nach Anspruch 8, wobei das Temperatursteuerungssystem so konfiguriert ist, dass es die Temperatur des Probenbereichs (540) aufrechterhält, wobei das Aufrechterhalten der Temperatur das Erwärmen des Probenbereichs (540) umfasst, und wobei das Aufrechterhalten der Temperatur das Kühlen des Probenbereichs (540) umfasst.

10. Abfragezelle (500) nach Anspruch 8, wobei das Temperatursteuerungssystem (109) einen Temperatursensor (740) umfasst, der mindestens ein Element aus einer Gruppe bestehend aus: mindestens einem Thermoelement, mindestens einem optischen Sensor, mindestens einem Infrarotsensor oder Kombinationen davon umfasst.

11. Abfragezelle (500) nach Anspruch 8, wobei das Temperatursteuerungssystem (109) eine thermoelektrische oder Peltier-Vorrichtung umfasst.

12. Abfragezelle (500) nach Anspruch 1, wobei die Abfragezelle (500) eine Energiequelle (10C) umfasst, und wobei die Energiequelle mindestens eine Batterie umfasst.

13. Abfragezelle (500) nach Anspruch 1, wobei die Energiequelle einen Auflademechanismus umfasst, der so konstruiert und angeordnet ist, dass er die Rotationsenergie der Abfragezelle (500) in elektrischen Strom umwandelt.

14. Abfragezelle (500) nach Anspruch 13, wobei die Stromquelle mindestens eine galvanische Zelle (850) umfasst, wobei die mindestens eine galvanische Zelle zwei Elektroden umfasst, die durch eine Elektrolytlösung in Form einer Konzentrationszelle getrennt sind, und wobei der Strom durch eine Elektrolytkonzentrationsdifferenz in der Elektrolytlösung angetrieben wird, die durch Zentrifugation induziert wird.

15. Abfragezelle (500) nach Anspruch 1, wobei die Abfragezelle (500) eine Energiequelle umfasst, wobei die Energiequelle mindestens eine Batterie umfasst, und wobei die mindestens eine Batterie mindestens eine galvanische Zelle (850) ist und durch einen radioisotopischen Zerfall (860) betrieben wird.

## Revendications

1. Cellule d'interrogation (500), comprenant :
une source de rayonnement électromagnétique (524) au niveau d'une première position, la source de rayonnement électromagnétique étant configurée pour émettre un rayonnement électromagnétique (524) au niveau d'une ou de plusieurs longueurs d'onde ;
une région d'échantillon (540) ; et
un détecteur (525) au niveau d'une seconde position, le détecteur étant configuré pour recevoir un rayonnement électromagnétique qui traverse au moins une partie de la région d'échantillon,
**caractérisé en ce que** la cellule d'interrogation (500) est dimensionnée pour être positionnée dans une cavité de rotor d'un rotor centrifuge (127).

2. Cellule d'interrogation (500) selon la revendication 1, dans laquelle la région d'échantillon (540) comprend une forme de secteur dans laquelle la région d'échantillon comprend une chambre d'échantillon (506) et dans laquelle la chambre d'échantillon (506) comprend une doublure interne amovible.

3. Cellule d'interrogation (500) selon la revendication 1, dans laquelle un système est configuré pour fournir des informations spectroscopiques de la région d'échantillon (540) sur la base des informations stockées collectées par le détecteur (525) et dans laquelle le système est configuré pour fournir au moins un type d'informations spectroscopiques à partir d'un groupe constitué de : données d'image hyperspectrales, images de Schlieren, images de fluorescence, ou données d'émission de fluorescence quantitative comprenant un agencement spatial de la région d'échantillon (540), ou des combinaisons de celles-ci.

4. Cellule d'interrogation (500) selon la revendication 3, comprenant en outre au moins un composant optiquement couplé à la source de rayonnement électromagnétique (524) et au détecteur (525), l'au moins un composant étant au moins un composant sélectionné à partir d'un groupe constitué de : au moins un interféromètre de Fabry-Perot, un ou plusieurs filtres optiques, une ou plusieurs lentilles optiques, un ou plusieurs miroirs, un ou plusieurs diffuseurs optiques, et un ou plusieurs collimateurs optiques, ou des combinaisons de ceux-ci.

5. Cellule d'interrogation (500) selon la revendication 4, dans laquelle un ou plusieurs des un ou plusieurs collimateurs optiques comprend/comprennent au moins un cristal photonique à collimation automatique.

6. Cellule d'interrogation (500) selon la revendication 4, dans laquelle un ou plusieurs des un ou plusieurs collimateurs optiques comprend/comprennent au moins une microlentille de Fresnel.

7. Cellule d'interrogation (500) selon la revendication 3, comprenant en outre un système diviseur de faisceau construit et agencé pour diriger un rayonnement électromagnétique (524) à partir de la source de rayonnement électromagnétique (524) vers une pluralité d'emplacements et dans laquelle le diviseur de faisceau comprend au moins un composant issu d'un groupe constitué de : au moins un miroir, au moins un filtre, au moins une lentille, ou des combinaisons de ceux-ci.

8. Cellule d'interrogation (500) selon la revendication 1, comprenant en outre un système de régulation de température (109) configuré pour modifier une température de la région d'échantillon (540).

9. Cellule d'interrogation (500) selon la revendication 8, dans laquelle le système de régulation de température est configuré pour maintenir la température de la région d'échantillon (540), dans laquelle le maintien de la température comprend le chauffage de la région d'échantillon (540), et dans laquelle le maintien de la température comprend le refroidissement de la région d'échantillon (540).

10. Cellule d'interrogation (500) selon la revendication 8, dans laquelle le système de régulation de température (109) comprend un capteur de température (740) qui comprend au moins un article d'un groupe constitué de : au moins un thermocouple, au moins un capteur optique, au moins un capteur infrarouge, ou des combinaisons de ceux-ci.

11. Cellule d'interrogation (500) selon la revendication 8, dans laquelle le système de régulation de température (109) comprend un dispositif thermoélectrique ou de Peltier.

12. Cellule d'interrogation (500) selon la revendication 1, dans laquelle la cellule d'interrogation (500) comprend une source d'alimentation (10C), et dans laquelle la source d'alimentation comprend au moins une batterie.

13. Cellule d'interrogation (500) selon la revendication 1, dans laquelle la source d'alimentation comprend un mécanisme de recharge construit et agencé pour convertir une énergie de rotation de la cellule d'interrogation (500) en courant électrique.

14. Cellule d'interrogation (500) selon la revendication 13, dans laquelle la source d'alimentation comprend au moins une cellule voltaïque (850), dans laquelle l'au moins une cellule voltaïque comprend deux électrodes séparées par une solution électrolytique, sous la forme d'une cellule de concentration, et dans laquelle le courant est entraîné par une différence de concentration d'électrolytes dans la solution électrolytique qui est induite par centrifugation.

15. Cellule d'interrogation (500) selon la revendication 1, dans laquelle la cellule d'interrogation (500) comprend une source d'alimentation, dans laquelle la source d'alimentation comprend au moins une batterie, et dans laquelle l'au moins une batterie est au moins une cellule voltaïque (850) et est entraînée par une décroissance radio-isotopique (860).
